(19) Europäisches Patentamt
European Patent Office
Office européen des brevets

(11) Veröffentlichungsnummer : **0 009 552**
**B1**

(12) # EUROPÄISCHE PATENTSCHRIFT

(45) Veröffentlichungstag der Patentschrift : **05.10.83**

(21) Anmeldenummer : **79102470.6**

(22) Anmeldetag : **16.07.79**

(51) Int. Cl.³ : **C 07 H 7/02**, C 08 G 65/28, C 07 H 1/00, C 08 B 31/00, C 08 B 37/00, C 08 B 15/00, C 07 H 15/04, C 08 G 63/12, C 08 G 18/32, C 08 G 18/48

(54) **In alpha-Stellung methylolierte Mono- und Oligosaccharide, Verfahren zu ihrer Herstellung und ihre Verwendung.**

(30) Priorität : **28.07.78 DE 2833138**

(43) Veröffentlichungstag der Anmeldung : **16.04.80 Patentblatt 80/08**

(45) Bekanntmachung des Hinweises auf die Patenterteilung : **05.10.83 Patentblatt 83/40**

(84) Benannte Vertragsstaaten : **BE DE FR GB**

(56) Entgegenhaltungen :
**EP A 0 000 912**
**EP A 0 001 210**
**EP A 0 001 211**
**EP A 0 001 389**
**EP A 0 002 473**
**EP A 0 004 543**
**EP A 0 007 100**
**EP A 0 007 101**
**EP A 0 007 102**
**BE A 655 756**
**DE A 1 196 870**
**DE A 2 714 084**
**FR A 1 443 898**
**FR A 2 363 537**
**FR A 2 363 596**
**FR A 2 390 412**

**TETRAHEDRON LETTERS, Nr. 19, 1978, Pergamon Press Ltd, Oxford, GB PAKTSUN HO : « Branched-chain sugars, I. Reaction between furanoses and formaldehyde » A synthesis of D-Hamamelose (1) », Seiten 1623-1626**

(73) Patentinhaber : **BAYER AG**
**Zentralbereich Patente, Marken und Lizenzen**
**D-5090 Leverkusen 1, Bayerwerk (DE)**

(72) Erfinder : **Wagner, Kuno, Dr.**
**Am Kiesberg 8**
**D-5090 Leverkusen (DE)**

(56) Entgegenhaltungen :
**CHEMICAL ABSTRACTS, Band 78, Nr. 15, 16. April 1973, Seite 505, Zusammenfassung Nr. 97899w, Columbus, Ohio, US, GLOTOVA, Yu. et al. : « Compounds of carbohydrates with formaldehyde »**

**TETRAHEDRON LETTERS, Nr. 32, 1978, Pergamon Press Ltd, Oxford, GB, J.C. DEPEZAY et al. : « Sucres branchés : Synthèse par aldolisation dirigée des désoxy-2 méthylène-2C d-érythro et D-thréo pentoses », Seiten 2865-2868**

**TETRAHEDRON LETTERS, Nr. 32, 1978, Pergamon Press Ltd, Oxford, GB, J.C. DEPEZAY et al. : « Sucres branchés : Epoxydation stéréospécifique de désoxy-2 méthylène-2C D-pentoses. Synthèse de méthyl glycosides de l'épihamamélose et de l'hamamélose », Seiten 2869-2872**

**EP 0 009 552 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

In α-Stellung methylolierte Mono- und Oligosaccharide, Verfahren zu ihrer Herstellung und ihre Verwendung

Die vorliegende Erfindung betrifft bislang unbekannte, in α- und/oder α'-Stellung zur Carbonylgruppe (bzw. durch Cyclohalbacetalbildung verkappten Carbonylgruppe) C-methylolierte $C_6$-Mono- und/oder Oligosaccharide mit einem Molekulargewicht bis 2 000, wobei erfindungsgemäß unter Mono- und Oligosacchariden solche zu verstehen sind, welche Fehling'sche Lösung reduzieren. Die vorliegende Erfindung betrifft weiterhin ein Verfahren zur Herstellung der neuen in α-Stellung zur Carbonylgruppe methylolierten $C_6$-Zucker sowie die Verwendung der neuen erfindungsgemäßen Stoffe. Dies betrifft sowohl natürliche Zucker als auch Derivate von natürlichen Zuckern, wie z. B. Aminozucker oder Monosaccharide, Disaccharide, Trisaccharide und oligomere Polysaccharide, welche durch partielle saure oder enzymatische Hydrolyse natürliche Polysaccharide, z. B. von Stärke (d. h. Amylose und Amylopektin), Cellulose, Inulin, Hemicellulosen und Glykogen oder aus Holz (d. h. Lignin enthaltenden Cellulosen) erhalten werden.

Es ist bekannt, daß die verschiedensten, Cyclohalbacetal-Endgruppen aufweisenden und Fehling'sche Lösung reduzierenden Monosaccharide wie Traubenzucker (d. h. α- und β-Glucose) und Fructose, Disacharide wie Maltose, Lactose, Cellobiose etc., sowie Tri- und Oligosaccharide in Gegenwart von Alkalien oder organischen Basen tiefgreifenden Umlagerungs- und Zersetzungsreaktionen unterliegen (vgl. Langenbeck, Lehrbuch der Org. Chemie (1952), S. 258 ff). Art und Ausmaß dieser oft unter starker Farbvertiefung ablaufenden Umlagerungs-, Karamelisierungs-, Dehydratisierungs- und Polymerisationsreaktionen, Vorstufen von Carbonisierungsreaktionen, sowie Cannizzaro-Reaktionen der Aldo- und Ketozucker untereinander im basischen Milieu sind weitgehend unbekannt, da die extrem schwierige Isolierung der Reaktions- und Zersetzungsprodukte und ihre ausschließliche Löslichkeit in Wasser die Anwendung aussagekräftiger spektroskopischer Methoden stark einschränkt oder ausschließt.

Es ist ferner aus eigenen Untersuchungen bekannt, daß die oben beispielhaft angeführten Zucker in wäßriger Lösung mit Formaldehyd bevorzugt im pH-Bereich größer als 7 je nach Menge des Formaldehyds penta- bis octafunktionelle Zuckerhalbacetale der schematischen Struktur

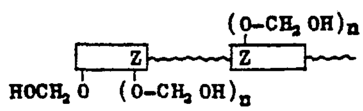

bilden, die in wäßriger Lösung wiederum im Gleichgewicht mit Methylenglykol ($HO—CH_2—OH$) stehen. Durch Wasserentfernung bei vermindertem Druck (z. B. 0,06 bis 15 Torr) können die Zucker-Polyhalbacetale rein erhalten werden. Diese Halbacetale spalten jedoch schon bei der Hydrolyse im neutralen Medium Formaldehyd extrem leicht wieder ab, ebenso in der Wärme. Durch Entwässerung bei pH-Werten unter 5,5 können unter Wasserabspaltung in die Zucker cyclische und kettenverlängernde oder kettenverzweigende bzw. vernetzende, thermisch und auch gegen alkalische Hydrolyse stabile Ganzacetalgruppen eingeführt werden, die jedoch in sauren pH-Bereichen wieder leicht abhydrolysiert werden.

In Tetrahedron L. No.19, S. 1623 (1978) werden hydroxymethylierte $C_4$- und $C_5$-Zucker beschrieben, die durch Umsetzung ihrer Acetalform mit Acetaldehyd bzw. Aceton als Schutzgruppe mit Formaldehyd und Kaliumcarbonat in Methanol in zweitägiger Reaktion entstanden.

In FR 2 363 537 und FR 2 363 596 wird gezeigt, daß bei der als Formose-Reaktion bekannten Selbstkondensation des Formaldehyds am Ende der Reaktion zum Abreagieren des Formaldehyds auch Nebenreaktionen auftreten, bei denen in geringem Maße C-Hydroxymethylierungen vorkommen. Es handelt sich hierbei um Nebenreaktionen an synthetischen Sacchariden, bei denen solche Reaktionen offenbar wegen der beim Aufbau der Formosemoleküle noch vorhandenen offenkettigen Struktur und damit vorhandener aktivierter Aldehydgruppe möglich ist. Die Hauptmenge der Formosemoleküle erfahren eine solche C-Hydroxymethylierung nicht. Ebenso wurde nicht gefunden, daß die beispielsweise als Co-Katalysator mitverwendbare Glucose hydroxymethyliert wird. Dies letztere liegt wahrscheinlich an der im alkalischen Bereich sehr stabilen Cycloacetalbildung.

Überraschenderweise wurde nun gefunden, daß es möglich ist, in Gegenwart von bevorzugt katalytischen Mengen an basischen Verbindungen der verschiedensten Art, im pH-Bereich 7,4 bis 11, bevorzugt im pH-Bereich 8 bis 9 und besonders bevorzugt bei pH = 8,4-8,6, natürliche, optisch aktive Zucker und deren Derivate wie α- und β-D-Glucose, Fructose, Aminoglucose, Invertzucker, Maltose, Lactose, Cellobiose, Trisaccharide und höhermolekulare Oligosaccharide, sofern diese Zucker Cyclohalbacetalendgruppen besitzen, d. h. in der Lage sind, Fehling'sche Lösung zu reduzieren, in α-Stellung zur Carbonylgruppe ohne Dehydratisierungs-, Isomerisierungs- und andere stark farbvertiefend wirkende Reaktionen an ihren α-C-Atomen zu methylolieren, wobei thermisch und hydrolytisch äußerst beständige neue C-Bindungen in die Zucker eingeführt werden. Vorteilhafterweise wird dabei bei Aldozuckern die Funktionalität an primären Hydroxylgruppen um eine Einheit erhöht, während bei Ketozuckern die Zahl der primären Hydroxylgruppen je nach Position der Ketogruppe um 2 oder sogar 3 vermehrt werden kann.

Gegenstand der Erfindung sind somit Umsetzungsprodukte natürlicher, Fehling'sche Lösung reduzierender $C_6$-Zucker und ihrer Oligomeren mit einem Molekulargewicht bis zu 2 000, welche dadurch gekennzeichnet sind, daß sie in α- und/oder α'-Stellung zur Carbonylgruppe bzw. Cyclohalbacetalgruppe des $C_6$-Zuckers mindestens eine, das Kohlenstoffgerüst des $C_6$-Zuckers verzweigende, Methylolgruppe aufweisen.

Wie schon erwähnt, sollen erfindungsgemäß unter « natürlichen $C_6$-Zuckern » nicht nur native Mono-, Di- und Oligosaccharide sondern auch Fehling'sche Lösung reduzierende Hydrolysate nativer Di- und Polysaccharide verstanden werden.

Gegenstand der Erfindung ist auch ein Verfahren zur Herstellung der neuen Umsetzungsprodukte von $C_6$-Zuckern und ihrer Oligomeren mit einem Molekulargewicht bis zu 2 000, welches dadurch gekennzeichnet ist, daß man ein Fehling'sche Lösung reduzierendes Derivat eines natürlichen $C_6$-Zuckers gegebenenfalls in Anwesenheit von Wasser und/oder ein- oder mehrwertigen Alkoholen mit einem Molekulargewicht zwischen 32 und 10 000, bei einer Temperatur von 40-110 °C, vorzugsweise 55-90 °C, besonders bevorzugt 80-85 °C, und bei einem pH-Wert von 7,4 bis 11, vorzugsweise 8-9, mit 0,05 bis 10 Mol, vorzugsweise 0,2 bis 5 Mol, besonders bevorzugt 1,0 bis 1,5 Mol und insbesondere 1,18 bis 1,20 Mol., bezogen auf die Äquivalente an zur Carbonylgruppe bzw. Cyclohalbacetalgruppe des Zuckers α- und α'-ständigen Wasserstoffatomen, an Formaldehyd umsetzt, wobei bevorzugt in Gegenwart von 0,01 bis 0,34 Äquivalenten, besonders bevorzugt 0,04 bis 0,06 Äquivalenten, bezogen auf α- und α'-ständige Wasserstoffatome, einer vorzugsweise organischen Base, sowie bevorzugt in Abwesenheit von Metallionen, besonders bevorzugt in Abwesenheit von mehrwertigen Metallionen, ganz besonders bevorzugt in Abwesenheit von zweiwertigen Metallionen, gearbeitet wird und wobei bevorzugt die Reaktion bei einem Restformaldehydgehalt von 0,3-0,9 Gew.-% im Reaktionsgemisch durch Ansäuern und/oder Kühlen abgebrochen wird.

Das erfindungsgemäße Verfahren erschließt eine neue Stoffklasse interessanter, definiert verzweigter, nativer Mono- und Oligosaccharide. Es ermöglicht die Modifizierung der verschiedensten nativen Zucker, Aldosen und Ketosen und ihrer D, L-Racemate. Zum Beispiel gelingt es erfindungsgemäß, in die folgenden Aldosen an den durch Pfeile gekennzeichneten α-C-Atomen eine $CH_2$—OH-Gruppe einzuführen :

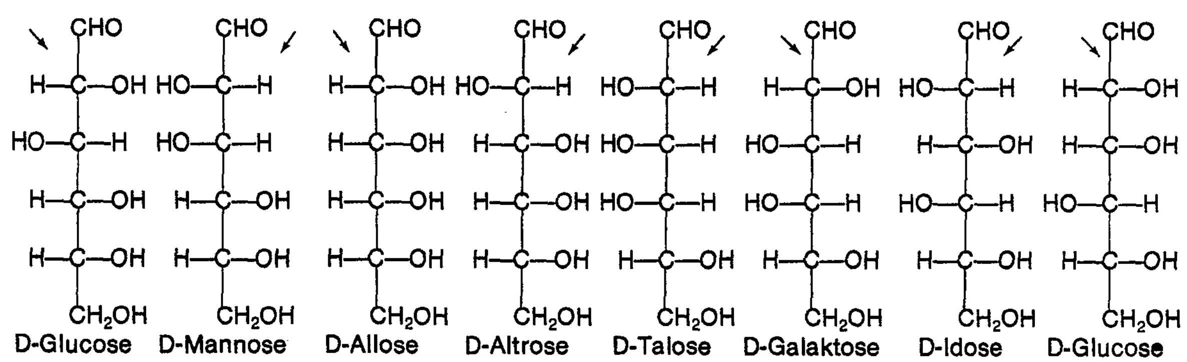

D-Glucose D-Mannose D-Allose D-Altrose D-Talose D-Galaktose D-Idose D-Glucose

Die erfindungsgemäß in das Zuckermolekül eingeführte zusätzliche primäre Hydroxylgruppe hat im wesentlichen gleiche Reaktivität wie die bereits vorhandene und ist den verschiedenartigsten Modifizierungsreaktionen zugänglich, wie sie weiter unten näher beschrieben werden.

Es ist wahrscheinlich, daß in den erfindungsgemäß modifizierten Zuckern Ringschluß aus der offenkettigen zur Cyclohalbacetalform eintritt, wie es für den Fall der α-C-methylolierten α-D-Glucose und β-D-Glucose in den nachstehenden Formeln gezeigt ist :

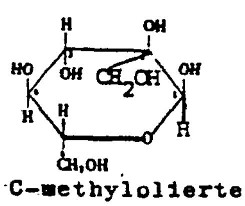

C-methylolierte α-D-Glucose

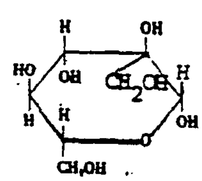

C-methylolierte β-D-Glucose

In entsprechender Weise werden auch aus nativen Disacchariden nach Durchführung der α-C-Methylolierung neue verzweigte Disaccharide mit zwei gleichwertig reagierenden primären Hydroxylgruppen erhalten. Bei Oligosacchariden mit Zuckerresten in glykosidischer Bindung, z. B. in Maisstärke-, Kartoffelstärke- oder Getreide-Hydrolysaten oder in anderen enzymatisch hydrolysierten Polysacchariden wird hierdurch die Zahl der $CH_2OH$-Gruppen stets um 1 erhöht.

Im Falle der Ketosen ermöglicht das erfindungsgemäße Verfahren die Einführung von bis zu

3 Methylolgruppen, da sowohl die α- als auch die α'-Kohlenstoffatome in den Ketosen für die Addition des Formaldehyds ausreichend aktiviert sind, wie es im folgenden für D-Fructose und D-Sorbose beispielhaft gezeigt wird :

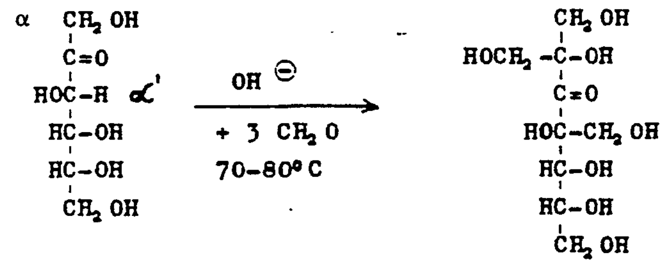


D-Fructose                α,α'-trimethylolierte Fructose

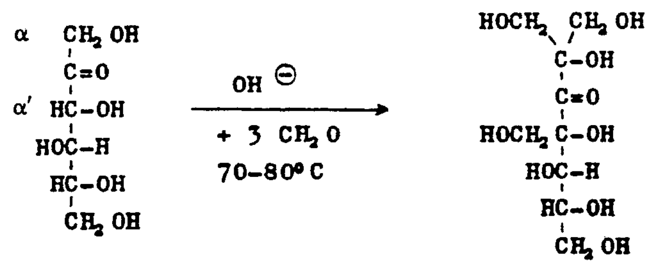


D-Sorbose                α,α'-trimethylolierte Sorbose

Von besonderem Vorteil ist, daß durch die erfindungsgemäße Einführung mindestens einer Methylolgruppe eine Verflüssigung der normalerweise kristallinen Zucker erreicht wird.

Die überraschend leicht in hoher Ausbeute (94 bis 96 %) ablaufende neue Additionsreaktion ist für den Fachmann unerwartet, da bisher bekannt war, daß die Cyclohalbacetale der natürlichen Zucker, z. B.

der Glucose                der Fructose

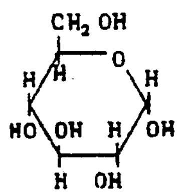

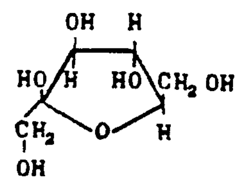

bei basischen pH-Werten (im Gegensatz zu dem für das erfindungsgemäße Verfahren ungeeigneten sauren pH-Bereich) sehr beständig sind und daher angenommen werden mußte, daß die obengenannten Cyclohalbacetalstrukturen, infolge der nicht ausreichenden Aktivierung der α- bzw. α'-ständigen C-Atome für eine Additionsreaktion des Formaldehyds im Sinne einer Aldolkondensation, nicht reagieren würden.

Überraschenderweise scheint aber trotzdem die im Gleichgewicht vorhandene (sehr geringe) Menge der offenkettigen Struktur ausreichend zu sein, um die Methylolierungsreaktion zu ermöglichen. Für den Fall der D-Glucose und ihrer optisch inaktiven Racemate kann der Reaktionsverlauf durch das folgende Reaktionsschema dargestellt werden :

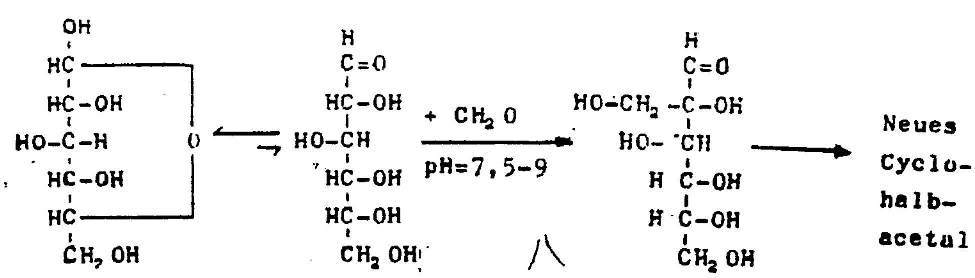


D-Glucose                (A)

In welchem Maße bei der erfindungsgemäßen Additionsreaktion auch der optische Antipode, also die L-Form zu (A), entsteht, ist zur Zeit noch nicht bekannt. Lösungen von (A) sind in Wasser optisch aktiv und besitzen einen Drehwert von $[\alpha_D] = +31°$. Die Entstehung des neuen α-C-methylolierten verzweigten $C_7$-Zuckers (A) aus Glucose und Formaldehyd in 95 %iger Ausbeute bzw. die Bildung des Cyclohalbacetals wurde durch Herstellung des chloroformlöslichen Hexaacetylderivates der Konstitution

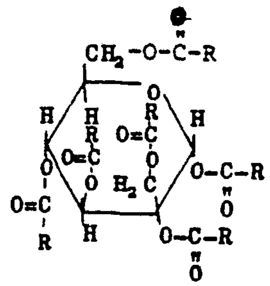

$R = CH_3$

durch Molekulargewichtsbestimmung und sein thermisches Verhalten sichergestellt.

Ein weiterer Beweis für die α-Verzweigung der erfindungsgemäß behandelten Zucker ist, daß die übliche Osazonbildung von Zuckern (Bindungen von 2 Mol Phenylhydrazin) nicht eintritt ; an die erfindungsgemäß modifizierten Zucker kann nur 1 Mol Phenylhydrazin unter Bildung des Hydrazons gebunden werden.

Wie schon erwähnt, sind der erfindungsgemäßen Methylolierungsreaktion nur solche Zucker bzw. Zuckerderivate zugänglich, welche Fehling'sche Lösung reduzieren.

In Lävoglucosan, einem Glucoseanhydrid, das durch Destillation von Glucose gemäß nachstehender Gleichung

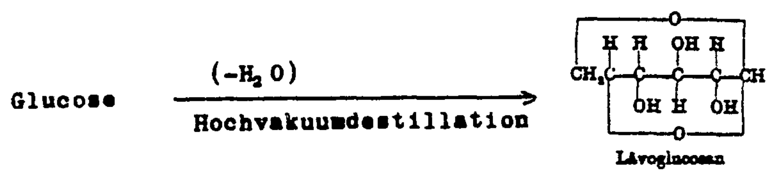


hergestellt werden kann, muß der erfindungsgemäßen Umsetzung daher erst der « Ganzacetalring » gespalten werden.

Das wirtschaftlich bedeutendste Disaccharid Saccharose, d. h. der Rohrzucker oder Rübenzucker der Konstitution

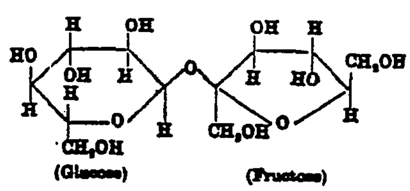

(Glucose) (Fructose)

ist im erfindungsgemäßen pH-Bereich und mit den erfindungsgemäßen Katalysatoren zunächst nicht α-C-methylolierbar. Nach in bekannter Weise ausführbarer saurer oder enzymatischer Hydrolyse zu Invertzucker (enzymatische Spaltung mit Saccharasen oder Invertasen oder z. B. $H^{\oplus}$-katalysierte heterogene Hydrolyse an sauren Ionenaustauschern) können jedoch pro Glucoseeinheit je eine neue Methylolgruppe und pro Fructoseeinheit maximal drei neue Methylolgruppen gebildet werden, so daß in der Gesamtbilanz pro Mol Rohrzucker 4 Mole Formaldehyd erfindungsgemäß als Methylolgruppen eingeführt werden können.

Da in der α-Methylolierbarkeit von Aldosen bzw. in der α,α'-Methylolierbarkeit von Ketosen ein allgemein gültiges Prinzip erkannt wurde, ist das erfindungsgemäße Verfahren auf beliebige monomere und polymere Zucker, auf beliebige D- oder L-Formen und deren Racematmischungen übertragbar.

Weitere wertvolle Ausgangssubstanzen aus der Reihe nativer hochmolekularer $C_6$-Polysaccharide, die primär Fehling'sche Lösung nicht reduzieren, jedoch nach saurer Hydrolyse zu Mono- und Oligosacchariden und Umstellung des pH-Wertes auf bevorzugt pH 8-9 erfindungsgemäß C-methylolierbar sind, sind die folgenden : Glykoproteide, Saponine, das Inulin $(C_6H_{10}O_3)_x$, das insbesondere in Knollen von Dahlien, in Artischocken, Topinamburknollen etc. vorhanden ist und ein hochpolymeres

Polysaccharid der Fructose darstellt, ferner Pektinstoffe (Polygalakturonsäuren), Naturstoffe wie Tannine, Chitin als Poly-N-Acetylglycosamin, stark Verzweigte Stärken wie Glykogen, ferner Amylopektine, Polymere aus D-Glucosamin-N-schwefelsäuren und D-Glucuronsäure (Heparin), sowie Chondroitinschwefelsäure, die — mit Proteinen verbunden — den Hauptbestandteil der Knorpelsubstanz darstellt und aus D-Glucuronsäure, N-Acetyl-D-Galactosamin und esterartig gebundener Schwefelsäure besteht. Interessante Ausgangssubstanzen, die primär hydrolysiert und dann α- und α'-C-methyloliert werden können, sind auch die verschiedensten in der belebten Natur vorkommenden Glycoside, d. h. Kondensationsprodukte von Mono- und Polysacchariden mit Alkoholen oder Phenolen, z. B. Glycoside der Flavonreihe, die in blauen und roten Blütenfarbstoffen und Beerenfarbstoffen vorkommen, die Klasse der sogenannten Digitalisglucoside, ferner die zuckerhaltigen Baukomponenten der Gerbstoffe aus der Tanningruppe, ferner Mucoitin-Schwefelsäure, dessen molekularer Aufbau der Chondroitinschwefelsäure ähnlich ist, das statt N-Acetyl-D-Galaktosamin jedoch N-Acetyl-D-Glucosamin enthält, ebenso hochmolekulare Hyaluronsäure, die im Molverhältnis 1 : 1 alternierend aus kondensierter D-Glucuronsäure und N-Acetyl-D-Glucosamin aufgebaut ist.

Der erfindungsgemäßen α-C- bzw. α,α'-C-Methylolierung sind auch Mono- und oligomere Polysaccharide mit anderen funktionellen Gruppen zugänglich, z. B. Zucker mit Carboxylgruppen wie D-Glucuronsäure

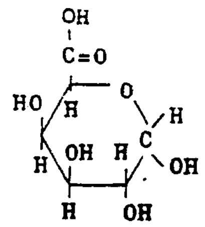

ihre Ester, Alkali- und Ammoniumsalze, ferner N-Acetylglucosamin

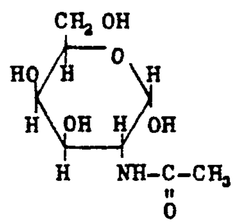

und Oligomere des N-Acetylglucosamins.

Als weitere Saccharid-Derivate, die der erfindungsgemäßen Reaktion zugänglich sind, sind zu nennen Natriumsalze der Gluco-pyranose-6-phosphorsäureester und Fructo-furanose-6-phosphorsäureester und ferner partiell (statistisch in 4, 5 oder 6-Stellung) acetalisierte oder ketalisierte Hexosen, partiell methylierte oder acetylierte Hexosen und ihre Stereoisomeren, sofern die α-Wasserstoffatome zur Carbonylgruppe in den Zucker-Derivaten noch vorhanden sind.

Wie gefunden wurde, können insbesondere sehr hochmolekulare Polysaccharide wie Cellulose, Maisstärke, Kartoffelstärke, Getreidestärke, Amylopektin, Inulin, Chitin, Chondroitinschwefelsäure etc., aber auch die wichtigsten Disaccharide wie Rohr- und Rübenzucker, ferner Reservepolysaccharide der Pflanzen, Hemicellulosen, Glycogen etc., die Fehling'sche Lösung nicht reduzieren, leicht in Form eines Eintopfverfahrens der erfindungsgemäßen α- bzw. α,α'-Additionsreaktionen des Formaldehyds zu verzweigten Zuckern zugänglich gemacht werden, wenn die vorgenannten Produkte in einer ersten Stufe sauer oder enzymatisch (fermentativ) hydrolysiert und in $C_6$-Oligosaccharide abgebaut werden, wodurch Fehling'sche Lösung reduzierende $C_6$-Saccharide und $C_6$-Oligosaccharide erhalten werden. Nach der Neutralisation sowie gegebenenfalls Entfernung der Anionen an Austauschersäulen und gegebenenfalls Entfernung von anderen Zellinhaltsstoffen, wie abgebauten und hydrolysierten Proteinen, sind die so erhaltenen, Fehling'sche Lösung reduzierenden Zuckergemische, die gegebenenfalls noch Aminosäuren und lösliche Oligopeptide gelöst enthalten können, mit Formaldehyd oder Formaldehyd enthaltenden Synthesegasen in einer zweiten Stufe erfindungsgemäß α- bzw. α,α'-C-methylolierbar.

Die Hydrolyse der Oligo- und Polysaccharide kann dabei in Form der bekannten Verfahren der « Holzverzuckerung » nach Bergius bzw. Willstätter und Zechmeister bei tiefen Temperaturen (z. B. mit hochkonz. Salzsäure) oder bei erhöhten Temperaturen (z. B. mit sehr verdünnter Schwefelsäure bei Normal- oder erhöhtem Druck bei 100 bis 110 °C) nach dem Verfahren von Scholler-Tornesch (vgl. Langenbeck, Lehrbuch der Org. Chemie 11/12 Auflage, S. 291) durchgeführt werden. Die gleichen Verfahren können auf den hydrolytischen Abbau von Bäckereihefen, zur Gärung verwendeter Hefearten, eiweißreicher Nährhefen etc. angewendet werden, um die zuckerhaltigen Zellinhaltsstoffe in Freiheit zu setzen.

Von erheblichem Interesse ist, wie gefunden wurde, daß auch Cyanhydrine, d. h. Anlagerungsprodukte von Blausäure an Aldosen und Ketosen der idealisierten Konstitution

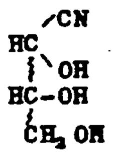 bzw. 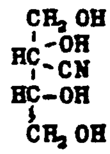

der erfindungsgemäßen Reaktion zugänglich sind, wobei eine partielle Abspaltung der Blausäure unter Bildung von Glykolnitril HO—$CH_2CN$ aus Formaldehyd und Blausäure erfolgt.

Von Interesse und überraschend ist, daß die aus Glucose und Fructose erhaltenen verzweigten $C_7$-$C_9$-Zucker mit üblichen Hefepräparaten vollständig vergärbar sind und die Geschwindigkeit der Äthanolbildung gemäß der Grundgleichung der Gärung

$$C_6H_{12}O_6 \quad \text{Hefenzyme} \quad 2C_2H_5OH + 2\,CO_2$$

nur geringfügig vermindert wird.

Bevorzugte Ausgangssubstanzen für das erfindungsgemäße Verfahren sind :

Naturprodukte wie α- und β-D-Glucose (Traubenzucker) ; sauer hydrolisierter oder enzymatisch hydrolysierter Rohrzucker (Invertzucker), hauptsächlich bestehend aus 1 Mol Glucose und 1 Mol Fructose ; Disaccharide wie Maltose, Lactose und Cellobiose ; handelsübliche hydrolysierte oder enzymatisch abgebaute Maisstärke und Kartoffelstärke in Form sogenannter Isosirupe ; natürliche Invertzucker, wie sie in den Honigsorten beliebiger Provenienz vorliegen ; sauer hydrolysierte Cellulose ; Hydrolyseprodukte des Inulins, die besonders reich an Fructose sind ; native Zucker, die mit geeigneten Aminen die Amadori-Umlagerung, die Heyns-Umlagerung, Lobry de Bruyn-Umlagerung oder Maillard-Reaktionen (vgl. Advances in Protein Chemistry, Volume 29, 1975, S. 185-188, Academic Press) eingegangen sind ; ferner Lignin enthaltende hydrolytisch abgebaute Oligopolysaccharide und Glucosehaltige Zucker der Holzverzuckerung, wobei das Lignin als Polymeres des Coniferylalkohols

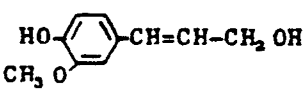

vorliegt. Da im Polymeren pro $C_{10}$-Monomereneinheit etwa 1 Äquivalent an aliphatischen Hydroxylgruppen und etwa 0,3 Äquivalente an aromatischen Hydroxylgruppen enthalten sind, entstehen aus derartigen Hydrolysaten bei der Durchführung des erfindungsgemäßen Verfahrens Mischungen der erfindungsgemäßen α-C-methylolierten Glucosen mit Halbacetalen des Lignins, die in o-Stellung zu den phenolischen Hydroxylgruppen C-methyloliert sind. Hierbei werden neue, modifizierte Lignine als Krümelige, amorphe, rotstichig-braune, thermoplastische Massen mit erhöhter Wasserquellbarkeit und Äthanol-Quellbarkeit erhalten.

Gegebenenfalls ist es auch möglich, das erfindungsgemäße Verfahren simultan mit den oben erwähnten Umlagerungen nach Amadori, Heyns und Lobry de Bruyn bzw. gekoppelt mit Maillard-Reaktionen ablaufen zu lassen, wobei interessante Gemische verschiedener verzweigter Zuckerderivate entstehen.

Als basische Katalysatoren für die erfindungsgemäße, einheitliche Einführung von Formaldehyd in die α-Stellung bzw. α'-Stellung von nativen $C_6$-Zuckern bzw. deren Derivate mit mindestens einer Cyclohalbacetalendgruppe oder offenkettigen Aldo- oder Ketofunktion sind zu nennen :

Hydroxide des Lithiums, Natriums und Kaliums ; Natrium-, Kalium- und Lithiumcarbonat ; Na- und K-Borat ; Lithium-, Natrium- und Kaliumcyanid ; Kaliumacetat ; Natrium- und Kaliumphenolat ; Lithium-, Natrium- und Kaliumsalze von peralkylierten Aminosäuren, bevorzugt des Dimethylaminoglyzins

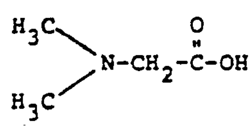 ;

Kronenäther-Komplexe von Alkaliatomen ; Natrium-, Kalium- und Lithiumsalze von phenolische Hydroxylgruppen enthaltenden Mannichbasen wie

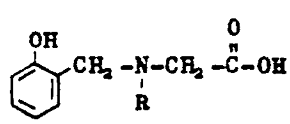

wobei R bevorzugt einen geradkettigen oder verzweigten aliphatischen Rest mit 1 bis 8 C-Atomen bedeutet ; ferner Alkalisalze von Tris-2,4,6-dimethylaminophenol.

Erfindungsgemäß bevorzugt sind jedoch organische Basen wie Trimethylamin, Triäthylamin, Tri-n-propylamin, Tri-n-butylamin, N,N-Dialkylbenzylamine mit 1 bis 5, vorzugsweise 1 oder 2, C-Atomen in den Alkylgruppen, N,N-Dimethylcyclohexylamin, N,N-Diäthylcyclohexylamin, N-Alkylpiperidine, N-N'-Dialkylpiperazine und N-Alkylmorpholine mit jeweils 1 bis 5, vorzugsweise 1 oder 2 C-Atomen in den Alkylgruppen, N-Phenylmorpholin, N-Benzylmorpholin, 1,2-Bis-morpholyl-äthan sowie Anlagerungsprodukte von Äthylenoxid und/oder Propylenoxid an Morpholin oder Piperazin mit einem Molekulargewicht bis etwa 2 000, vorzugsweise bis ca. 1 000. Diese letztgenannten Verbindungen sind ebenso wie andere, für die Polyurethanherstellung an sich bekannte, basische Polyäther (Alkoxylierungsprodukte von Ammoniak, primären und sekundären Mono- oder Polyaminen) erfindungsgemäß von Interesse, weil sie auch in höheren Konzentrationen in den Verfahrensprodukten verbleiben können und später bei der Verwendung der Produkte als Ausgangskomponente zur Herstellung von Polyurethankunststoffen einbaubare Katalysatoren für die Polyisocyanat-Polyadditionsreaktion darstellen.

Von Interesse als Katalysatoren sind auch bicyclische Amidine wie z. B.

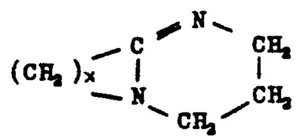

wobei X für 3, 4 oder 5 steht, ferner Endoäthylenpiperazin, permethyliertes Hexamethylendiamin, $N^1$, $N^2$, $N^3$-Trimethyl- und Triäthylhexahydrotriazin, permethylierte Alkylenpolyamine, N-formylierte Polyamine wie

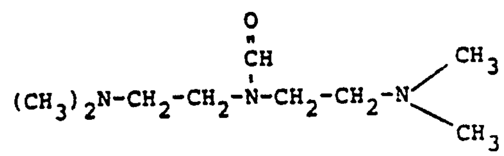

Pyridin, Chinolin und ihre methylsubstituierten Derivate allein oder in Mischung mit vorgenannten tert. Basen. Interessante Katalysatoren in dieser Gruppe sind auch Mannichbasen wie z. B.

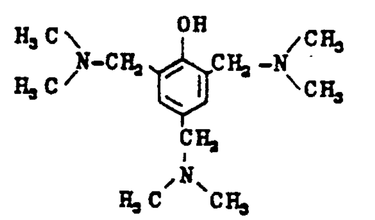

Eine weitere interessante Gruppe von Katalysatoren stellen auch bekannte Mono- und Polyepoxyde z. B. der Konstitution

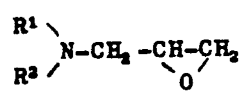

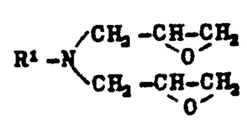

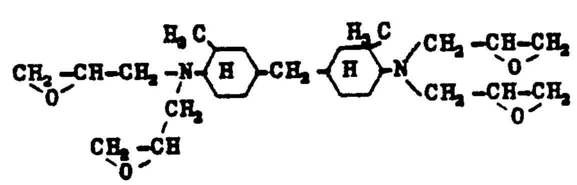

dar.

Beispiele für erfindungsgemäß besonders bevorzugte quartäre Ammoniumbasen sind 10 bis 20 %ige Lösungen von

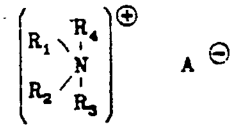

in Alkoholen wie Methanol oder Äthanol sowie

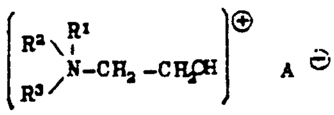

In den obigen Formeln stehen

$R^1$, $R^2$, $R^3$ und $R^4$ für Alkyl-, Cycloalkyl- oder Aralkylreste mit 1 bis 10 C-Atomen, vorzugsweise für Methyl, Äthyl, Cyclohexyl oder Benzyl, und

A für ein basisches Anion, vorzugsweise für ein Hydroxyl-, Acetat- oder Phenolation, besonders bevorzugt für ein Cyanidion.

Beispiele für besonders bevorzugte ternäre Sulfoniumbasen sind solche der Formeln

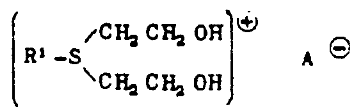

bzw.

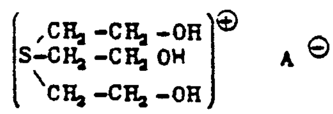

in denen $R^1$ und $A^{\ominus}$ die vorgenannte Bedeutung haben.

In der Reihe der anorganischen Katalysatoren sind Natriumhydroxyd, Natriumcyanid, Natriumphenolat und Natriummethylat besonders bevorzugt.

Unter den organischen basischen Katalysatoren sind besonders bevorzugt Dimethylcyclohexylamin, Triäthylamin, die Cyanide quartärer Ammoniumbasen wie Tetramethylammoniumcyanid und Tetraäthylammoniumcyanid und quartäre Ammoniumverbindungen betainartigen Charakters wie das Cholin, das aus Trimethylamin, Äthylenoxyd und 1 Mol Wasser in an sich bekannter Weise leicht hergestellt werden kann. Cholincyanid ist in dieser Gruppe besonders bevorzugt. Als unlösliche basische Katalysatoren sind auch stark basische Ionenaustauscher, z. B. solche mit Gruppierungen

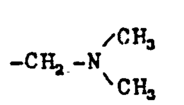 bzw. 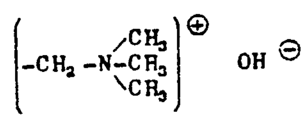

die an eine unlösliche Polystyrol-Matrix gebunden sind, verwendbar, jedoch verläuft die erfindungsgemäße Reaktion in diesem Fall um etwa den Faktor 50 langsamer, da die Diffusion der raumbeanspruchenden Zuckermoleküle an die basischen Zentren der Matrix relativ langsam abläuft.

Cyanide (sowohl von Alkalimetallen als auch von quartären Ammoniumverbindungen) sind dann besonders bevorzugt, wenn besonders reine und farblose verzweigte Zucker mit hoher Ausbeute hergestellt werden sollen.

Als Formaldehydquelle für die Durchführung des erfindungsgemäßen Verfahrens setzt man im allgemeinen 10-70 Gew.-%, vorzugsweise 20-65 Gew.-%, besonders bevorzugt 30 bis 50 Gew.-% Formaldehyd enthaltende wäßrige und/oder alkoholische Formalinlösungen und/oder Paraformaldehyddispersionen und/oder zur Transmethylolierung befähigte Verbindungen wie N-Methylol-caprolactam, N-Methylolpyrrolidon, N-methylolierte Harnstoffe und Thioharnstoffe, methylolierten N,N-Dimethylharnstoff, methyloliertes Dicyandiamid, methyloliertes Melanin, d. h. allgemein N-Methylolverbindungen von Aminoplastmonomeren ein. Gewünschtenfalls kann die erfindungsgemäße Reaktion auch in Gegenwart von Phenolen, Naphtholen, Bisphenol A, d. h. zur Phenoplastbildung befähigten Verbindungen durchgeführt werden, wobei Mischungen der erfindungsgemäßen Zucker mit nicht zur Transmethylolierung befähigten

Hydroxybenzylalkoholen bzw. Hydroxybenzylpolyalkoholen erhalten werden, die gegebenenfalls durch anschließendes Ansäuern auf pH = 2-4 leicht mit den alkoholischen Hydroxylgruppen der neuen Zuckerderivate verätherbar sind.

Selbstverständlich läßt sich das erfindungsgemäße Verfahren auch mit praktisch wasserfreien Halbacetalen des Formaldehyds mit Monoalkoholen wie Methanol, Äthanol und Propanol oder auch mit Halbacetalen von Polyalkoholen wie Äthylenglykol, Propylenglykol, Diäthylenglykol, Triäthylenglykol, Glyzerin, N-Methyldiäthanolamin, Dimethyläthanolamin oder Triäthanolamin durchführen, wobei im Falle der Verwendung basischer Alkohol-Halbacetale auf zusätzliche basische Katalysatoren verzichtet werden kann.

Besonders bevorzugte Formaldehydquellen sind auch die bei der großtechnischen Herstellung von Formaldehyd anfallenden heißen Synthesegase, die direkt für die α-C-Methylolierungsreaktion bzw. α,α'-C-Polymethylolierungsreaktion eingesetzt werden können. Man setzt zu diesem Zweck Lösungen der zu modifizierenden Zucker bei den erfindungsgemäß einzuhaltenden pH-Werten als Absorptionsflüssigkeiten für den Formaldehyd ein, indem man die Zuckerlösungen vorzugsweise zur Gegenstromwäsche der heißen Synthesegase verwendet und entweder gleichzeitig die Additionsreaktion, vorzugsweise bei ca. 50-95 °C und einem pH-Wert von 8-9, ablaufen läßt oder, falls die Absorptionsflüssigkeit keinen Katalysator enthält, die Methylolierungsreaktion durch nachträglichen Zusatz der oben beschriebenen Katalysatoren initiiert.

Für das erfindungsgemäße Verfahren sind beliebige Synthesegase einsetzbar, wie sie bei der technischen Herstellung von Formaldehyd anfallen. Beispiele für großtechnische Herstellungsverfahren von Formaldehyd sind die Dehydrierung von Methanol bzw. die Oxidation von Methanol an geeigneten Katalysatoren (beispielsweise Silber oder Eisenmolybdänoxid) in Gegenwart von Luft, gegebenenfalls Wasserdampf und Formaldehyd-Abgasen, sowie die Oxidation von Methan bzw. Äthylen oder höheren Olefinen oder Dimethyläther mit Luft oder Sauerstoff bzw. sauerstoffhaltigen Gasen an geeigneten Katalysatoren. Diese technischen Synthesegase enthalten im allgemeinen als Hauptbestandteil 20 bis 70 Vol.-% Stickstoff, 1 bis 20 Vol.-% Formaldehyd, 1 bis 10 Vol.-% Kohlendioxid sowie meist — je nach Herstellungsverfahren — auch größere Mengen an Wasserdampf, restlicher Luft, Kohlenmonoxid, Wasserstoff und Restmengen an Ausgangsprodukten oder auch Nebenprodukte wie z. B. Methanol, Methan, Äthylen, höhere Olefine, Methylformiat, Dimethyläther sowie Acetale und Halbacetale des Formaldehyds. Häufig neigen die Zucker in Anwesenheit von Sauerstoff zu Verbräunungsreaktionen; Sauerstoff-freie Synthesegase sind daher erfindungsgemäß bevorzugt.

Die bei der großtechnischen Herstellung von Formaldehyd anfallenden Synthesegase können jedoch im übrigen roh, d. h. ohne jede vorgeschaltete Reinigungsstufe, im erfindungsgemäßen Verfahren eingesetzt werden, was aus ökonomischen Gründen von besonderem Vorteil ist. Auch die üblicherweise in den Synthesegasen vorhandenen großen Mengen an Kohlendioxid stören die erfindungsgemäße Methylolierungsreaktion nicht.

Die erfindungsgemäße Verfahrensvariante, bei welcher Synthesegase als Formaldehydquelle dienen, kann sowohl bei Normaldruck als auch bei Überdruck als auch bei vermindertem Druck (mit abgeschreckten Synthesegasen) ausgeführt werden. Die Absorptionsflüssigkeit, bestehend aus einer Lösung des zu methylolierenden Zuckers, welche gegebenenfalls ca. 10 bis 60 Gew.-% der weiter unten beschriebenen Mono- und Polyalkohole mit einem Molekulargewicht von 32 bis 10 000 sowie gegebenenfalls 1 bis 50 Gew.-% der ebenfalls weiter unten beschriebenen Aminoplast- oder Phenoplastmonomeren, Phosphite und/oder α-methylolierbaren Aldehyde und Ketone enthält, wird in einer geeigneten Absorptionskolonne vorgelegt und das Synthesegas mit einer Temperatur von ca. 90 bis 250 °C, vorzugsweise 100 bis 140 °C, in die auf ca. 70 bis 110 °C, vorzugsweise 80 bis 85 °C, gehaltene Absorptionsflüssigkeit kontinuierlich oder diskontinuierlich eingeleitet.

Man kann das Wärmereservoir der Synthesegase (die bei ihrer Herstellung auf eine Temperatur von ca. 300 °C abgeschreckt anfallen) bzw. die Wärmemenge, die bei der Methylolierung frei wird, in energiesparender Weise zur teilweisen Entwässerung der Verfahrensprodukte ausnützen. Von großem Vorteil ist in diesem Zusammenhang die Schleppmittelwirkung der im Synthesegas vorhandenen großen Inertgasvolumina für Wasser. Die freiwerdende Wärmemenge kann aber auch an die verschiedensten Kreisläufe der Formaldehydherstellung geliefert werden, z. B. an die Verdampferkreisläufe für Methanol und/oder Wasser, so daß die anfallende Wärmeenergie optimal ausgenützt werden kann.

Bei der diskontinuierlichen Verfahrensvariante wird das Synthesegas durch eine Säule geleitet, welche mit der ruhenden Absorptionsflüssigkeit gefüllt ist. Um den Stoffaustausch zwischen den beiden Phasen zu beschleunigen, enthält dabei die Absorptionssäule zweckmäßigerweise Füllkörper mit großer Oberfläche der an sich bekannten Art wie z. B. Raschigringe, Sattelringe, Siebböden oder feinmaschige Drahtnetze. Das Synthesegas wird durch die Absorptionssäule bis zur Sättigung der Absorptionsflüssigkeit geleitet, d. h. bis am Kopf der Säule größere Mengen an Formaldehyd zusammen mit den Inertgasen austreten. Vorzugsweise enthält die Absorptionsflüssigkeit bereits den für das erfindungsgemäße Verfahren erforderlichen Katalysator, so daß die Methylolierungsreaktion schon während der Absorption des Formaldehyds beginnt. Der Vorteil dieser Arbeitsweise liegt darin, daß durch ein gegebenes Volumen von Absorptionsflüssigkeit eine größere Menge an Formaldehyd aufgenommen werden kann. Selbstverständlich ist es jedoch erfindungsgemäß möglich, die Synthesegase bis zur Sättigung in eine katalysatorfreie Absorptionsflüssigkeit einzuleiten und erst danach durch Zusatz des

Katalysators die Reaktion zu starten.

Besonders wirtschaftlich ist es jedoch, wie bereits erwähnt, das erfindungsgemäße Verfahren kontinuierlich zu gestalten. Man hält zu diesem Zweck einen Kreislauf der Absorptionsflüssigkeit aufrecht und führt die Absorptionsflüssigkeit zweckmäßigerweise im Gegenstrom den heißen Synthesegasen entgegen. Auch bei dieser bevorzugten Verfahrensvariante ist es von Vorteil, um den Stoffaustausch zu erleichtern, als Absorptionssäulen an sich bekannte Füllkörper-, Glockenboden-, Siebboden- oder Rieselfilmkolonnen einzusetzen ; selbstverständlich können auch Blasensäulen für das erfindungsgemäße Verfahren verwendet werden. Die durchschnittliche Verweilzeit der formaldehydhaltigen Synthesegase in den Absorptionskolonen liegt sowohl bei der kontinuierlichen als auch bei der diskontinuierlichen Variante des erfindungsgemäßen Verfahrens im allgemein zwischen 0,3 und 10 Sekunden, vorzugsweise zwischen 0,6 und 3 Sekunden.

Figur 1 zeigt in stark vereinfachter, schematischer Darstellung eine für die kontinuierliche Ausgestaltung des erfindungsgemäßen Verfahrens geeignete Apparatur : In die mit Absorptionsflüssigkeit gefüllte Absorptionskolonne A wird bei 1 das heiße, Formaldehyd enthaltende Synthesegas eingeleitet. Bei 2 können die Zuckerlösung bzw. Zusätze wie z. B. Katalysator, Base, Alkohole, aldolisierbare Aldehyde bzw. Ketone, Aminoplastbildner etc. eingespeist werden. Über die Pumpe B wird die Absorptionsflüssigkeit umgepumpt und dem Synthesegas im Gegenstrom entgegengeführt. Bei 3 verlassen die von Formaldehyd befreiten, Wasserdampf enthaltenden Gase die Absorptionskolonne. C stellt ein heiz-bzw. kühlbares Verweilzeitgefäß dar, in welchem die Methylolierungsreaktion ablaufen kann. Bei 4 wird laufend ein Teil des Reaktionsproduktes und Restmengen an Formaldehyd enthaltenden Absorptionsflüssigkeit abgenommen und durch ein weiteres Verweilzeitgefäß D geleitet, in welches bei 5 nochmals verschiedene Zusatzstoffe (Katalysator, Aminoplastbildner etc) zudosiert werden können. Bei 6 verläßt das Reaktionsprodukt die Apparatur.

Auch bei der kontinuierlichen Verfahrensweise kann, wie schon erwähnt, der basische Katalysator bereits der Absorptionsflüssigkeit zugesetzt werden, (ca. 0,01 bis 10 Gew.-%, vorzugsweise 0,3 bis 2 Gew.-%), so daß Absorption des Formaldehyds und Anlagerung des Formaldehyds an den Zucker simultan erfolgen ; es ist aber auch möglich, den Katalysator erst nach der Produktabnahme (also beispielsweise in Position 5 von Figur 1) zuzudosieren, so daß die Methylolierung im wesentlichen erst außerhalb des Kreislaufes erfolgt.

Enthält die Absorptionsflüssigkeit keinen basischen Katalysator, dann bilden sich zunächst im Gleichgewicht mit in Wasser gelöstem Formaldehyd die Halbacetale der in der Absorptionsflüssigkeit vorhandenen Hydroxylverbindungen, beispielsweise solche der nachstehenden Formel :

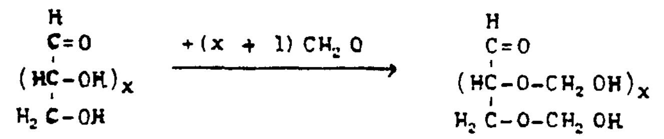


Da sich in Gegenwart von Wasser das Dissoziationsgleichgewicht zwischen diesen Halbacetalen und freiem Formaldehyd sehr schnell einstellt, wird auch aus derartigen Gemischen nach Zusatz des Katalysators bei 70 bis 110 °C, vorzugsweise 80 bis 85 °C, äußerst rasch der verzweigte Zucker gebildet.

Findet die Methylolierungsreaktion ganz oder teilweise außerhalb des Kreislaufs der Absorptionsflüssigkeit statt, dann kann man erfindungsgemäß die Reaktion beispielsweise in kontinuierlich laufenden Rührkesselkaskaden stattfinden lassen. Durch Variation der Verweilzeit in den einzelnen Rührkesseln der Kaskade läßt sich bei dieser Verfahrensvariante der Restformaldehydgehalt exakt einstellen. Es ist auch möglich, die erfindungsgemäße Reaktion des Formaldehyds statt in Rührkesselkaskaden in Reaktionsröhren, beispielsweise in Schlangenrohrreaktoren unter Druck (ca. 5 bis 150 bar, vorzugsweise 10 bis 70 bar) bei erhöhten Temperaturen (vorzugsweise 105 bis 140 °C) ablaufen zu lassen.

Im erfindungsgemäßen Verfahren kann die Menge an Formaldehyd in weiten Grenzen variiert werden. Im allgemeinen setzt man 0,05 bis 10 Mol, vorzugsweise 0,2 bis 5 Mol, besonders bevorzugt 1,0 bis 1,5 Mol, an Formaldehyd pro Äquivalent an α- oder α'-ständigen (d. h. durch eine Methylolgruppe substituierbaren) H-Atomen des Zuckers bzw. Zuckerderivats ein. Im Falle der Verwendung unterschüssiger Formaldehydmengen erhält man Gemische aus α- bzw. α'-methylolierten Zuckern und unmodifizierten Zuckern. Lösungen derartiger Gemische haben vorteilhafterweise gegenüber den Ausgangslösungen eine stark verminderte Viskosität und Kristallisationsneigung. Gemische der erfindungsgemäßen verzweigten Zucker mit unmodifizierten Zuckern (insbesondere Glucose und/oder Saccharose) im Gewichtsverhältnis 5 : 95 bis 95 : 5, vorzugsweise 20 : 80 bis 80 : 20, sind daher ebenfalls Gegenstand der vorliegenden Erfindung.

Setzt man im erfindungsgemäßen Verfahren hingegen einen großen Überschuß an Formaldehyd ein (z. B. die ca. 5- bis 6-fache der theoretisch für die Methylolierung benötigten Menge), so erhält man nach der Entwässerung des Reaktionsgemisches (vorzugsweise in einem Vakuum von ca. 0,05-16 Torr) Halbacetale der erfindungsgemäßen verzweigten Zucker, welche gegebenenfalls durch Ansäuern (pH 1-4) zu neuartigen, verzweigten oder vernetzten Ganzacetalen oder Polyacetalen umgesetzt werden

können.

Will man hingegen die erfindungsgemäßen verzweigten Zucker in besonders hoher Reinheit herstellen, dann wendet man bevorzugt 1,18 bis 1,20 Mol Formaldehyd pro Äquivalent an α- bzw. α'-ständigen Wasserstoffatomen des Zuckers an und bricht die Reaktion bei einem Restformaldehydgehalt im Reaktionsgemisch von ca. 0,3 bis 0,9 Gew.-% durch Kühlen und/oder Neutralisieren ab.

Das erfindungsgemäße Verfahren läßt sich diskontinuierlich oder kontinuierlich, bei vermindertem Druck oder bei Überdruck ausführen. Gemäß einer besonderen Variante läßt man die Methylolierungsreaktion in einer Rührkesselkaskade ablaufen. Durch Variation der Verweilzeit und des pH-Wertes in den einzelnen Rührkesseln läßt sich bei dieser Verfahrensvariante der Umsetzungsgrad in der Addition des Formaldehyds an die α- und α'-C-Atome der Zucker bzw. Zuckerderivate (bevorzugt Glucose, Fructose, Maltose, Lactose, Cellobiose und ihre Gemische, Invertzucker, Honigarten, « Isosirupe », hydrolysierter Rohrzucker, hydrolysierte Stärke und verzuckerte Cellulose) exakt einstellen. Auf diese Weise ist es daher ebenfalls möglich, auch nur partielle C-Methylolierungsreaktionen an einem nativen Zucker durchzuführen und z. B. Mischungen herzustellen, die aus einem Mol von α-methylolierter Glucose und einem Mol nichtumgesetzter Glucose bestehen, wodurch die Kristallisation der entwässerten Mischung extrem gestört wird und die Viskosität der wasserarmen Zuckermischungen stark gesenkt werden kann.

Besonders hervorzuheben ist, daß bei der erfindungsgemäßen pH-Führung und bei Verwendung der bevorzugten basischen Katalysatoren im erfindungsgemäßen Verfahren gekreuzte Cannizzaro-Reaktionen des Formaldehyds extrem stark zurückgedrängt werden und die Zucker ohne Dehydratisierungsreaktionen wie auch ohne nennenswerte gekreuzte Cannizzaro-Reaktionen oder Aldolkondensationen der Zucker untereinander überraschend in 95-98 %iger Ausbeute erhalten werden können.

Im allgemeinen erfordern die erfindungsgemäßen C-Methylolierungen keine Katalysatordesaktivierung, da die bevorzugt eingesetzten geringen Mengen an Katalysator meist gegen Ende der Reaktion bei einem Restformaldehydgehalt der neuen, verzweigten Zucker von ca. 0,3-0,9 Gew.-% durch kleine Mengen sich bildender Ameisensäure oder durch Zuckersäuren desaktiviert werden und daher gegen Ende der Reaktion der pH-Wert im allgemeinen auf 7-6,8 absinkt. Gewünschtenfalls können die Reaktionsprodukte jedoch an sauren und basischen Ionenaustauschern entsalzt werden.

Vorzugsweise wird erfindungsgemäß, wie schon erwähnt, in Abwesenheit von Metallionen (insbesondere mehrwertiger Metallionen), also mit organischen Basen als Katalysator gearbeitet. Mehrwertige Metallionen katalysieren nämlich die Kondensation des Formaldehyds zu Polyhydroxyaldehyden und -ketonen (sogenannte Formosesynthese). Führt man die erfindungsgemäße α,α'-C-Methylolierung mit überschüssigem Formaldehyd, bezogen auf alle Aldo- und Keto-Äquivalente des verwendeten nativen Zuckers oder Zuckerderivats in Gegenwart von Katalysatoren wie Calciumhydroxyd, Bleihydroxyd, Calciumoxid, Bleioxid, Calciumphenolat, Calciumcarbonat, Thalliumhydroxid etc. aus, so entstehen daher neben den erfindungsgemäßen α,α'-C-methylolierten Zuckern Gemische von $C_3$-$C_9$-Polyhydroxyaldehyden, Polyhydroxyketonen und Polyalkoholen (Formosen) in Form ihrer optisch inaktiven D,L-Racemate.

Die erfindungsgemäße Reaktion wird oft mit Vorteil in Gegenwart von niedermolekularen, ein- oder vorzugsweise mehrwertigen Alkoholen ausgeführt, um relativ niedrigviskose, gut fließende und leicht im Dünnschichtverdampfer entwässerbare Reaktionsprodukte zu erhalten. Auch höhermolekulare Polyhydroxylverbindungen (Molekulargewicht bis 10 000) können gegebenenfalls mitverwendet werden, insbesondere wenn mit formaldehydhaltigen Synthesegasen gearbeitet wird.

Geeignete Mono- und Polyalkohole sind beispielsweise Methanol, Äthanol, Propanol, Butanol, Amylalkohol, sowie die unten im Zusammenhang mit der Herstellung von Polyurethankunststoffen ausführlich beschriebenen Polyhydroxylverbindungen, insbesondere Äthylenglykol, Glycerin, Trimethylolpropan, Formite, Diäthylenglykol, Triäthylenglykol, Propandiol-(1,2), Propandiol-(1,3), Butandiol-(1,4), N-Methyldiäthanolamin, N-Äthyl-diäthanolamin, oxäthyliertes bzw. propoxyliertes Äthylendiamin, oxäthyliertes oder propoxyliertes Hydrazin oder substituiertes Hydrazin (z. B. N,N-Dimethyl- oder Diäthylhydrazin), sowie auch wasserunlösliche aber emulgierbare Polyalkohole wie Rizinusöl, Hexantriol und 2-Äthylhexandiol-(1,3) sowie Oxäthylierungs- und Propoxylierungsprodukte aller genannten Mono- und Polyalkohole. Die erfindungsgemäße Methylolierung von Zuckern kann auch in Gegenwart von weiteren C- bzw. N-methylolierbaren Verbindungen durchgeführt werden. Man erhält in diesem Falle besser fließfähige, leichter entwässerbare und mit Polyisocyanaten schneller reagierende Verfahrensprodukte, die sich zur Herstellung flammwidriger Polyurethankunststoffe eignen. Als derartige reaktive Zusätze kommen z. B. ganz allgemein primäre und/oder sekundäre Amine und/oder zur Aminoplast- bzw. Phenoplastbildung geeignete Stoffe bzw. deren Methylolierungsprodukte in Frage, wie sie beispielsweise in den DE-Offenlegungsschriften 2 324 134 und 2 639 254 beschrieben werden. Beispielhaft seien in diesem Zusammenhang genannt : Anilin, Harnstoff, symmetrisch oder asymmetrisch substituierte Harnstoffe wie N,N-Dimethyl (bzw. -Diäthyl oder -Dibutyl)-Harnstoff, Thioharnstoff, Dicyandiamid, Melamin, Oxamid, Äthylenharnstoff, ε-Caprolactam Pyrrolidon-(2)-, Acetylendiurein und die N-Methylolverbindungen all dieser Aminoplastmonomeren, Phenole und methylolierte Phenole.

Auch nicht zuckerartige, α- bzw. α'-methylolierbare Aldehyde und Ketone können im erfindungsgemäßen Verfahren mitverwendet werden.

Beispiele hierfür sind Acetaldehyd, Aceton, Propionaldehyd, Butyraldehyd, Isobutyraldehyd, Methyläthylketon, Cyclopentanon, Cyclohexanon, Mesityloxid, Isophoron, Acetophenon sowie deren Methylolderivate, wie sie durch basenkatalysierte teilweise Aldolisierung mit Formaldehyd an den zur Ketogruppe α-ständigen C-Atomen erhältlich sind. Beispiele hierfür sind die Verbindungen der Formeln :

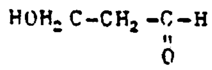

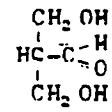

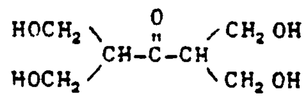

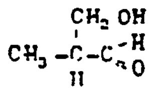

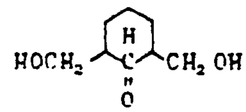

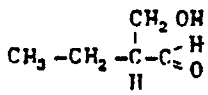

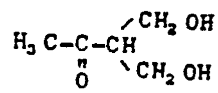

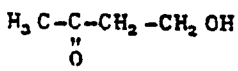

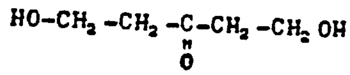

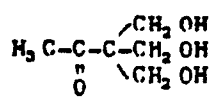

,

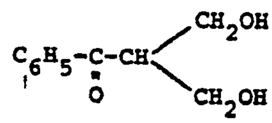

Auch Nebenprodukte der technischen Trimethylolpropanherstellung aus Buttersäurealdehyd und Formaldehyd wie z. B. 2-Äthylacrolein können mitverwendet werden, wobei z. B. 2-Äthylacrolein in Gegenwart von tert. Aminkatalysatoren wie Triisobutylamin in 2,2-Dimethylolalkanol übergeführt wird :

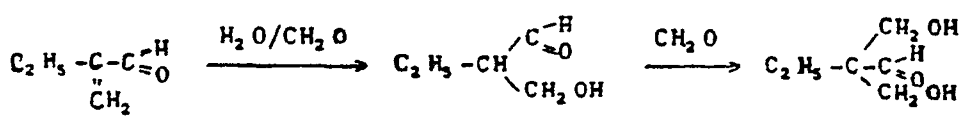


Auch diese methylolierten Aldehyde und Ketone führen zu einer vorteilhaften Erniedrigung der Viskosität der erfindungsgemäß hergestellten Zuckerderivate.

Erfindungsgemäß ist es auch möglich, bei der Methylolierungsreaktion Alkylphosphite wie z. B. Dimethylphosphit, Diäthylphosphit oder Triäthylphosphit mitzuverwenden.

Durch eine basenkatalysierte Reaktion mit dem Formaldehyd entstehen dabei α-Hydroxylmethylphosphonsäureester bzw. Umesterungsprodukte mit den Hydroxylgruppen der Zucker. Ähnlich reagieren auch andere CH-acide Verbindungen wie Malonsäureester oder Acetessigester. Insbesondere die mit Alkylphosphiten modifizierten Zucker stellen ein wertvolles Ausgangsmaterial für die Herstellung äußerst flammwidriger Polyurethanschaumstoffe dar.

Es ist als besonders überraschend zu bezeichnen, daß sich die erfindungsgemäßen verzweigten Zucker in den genannten Phosphiten lösen, während Glucose und andere Monosaccharide, aber auch Rohrzucker, in den Phosphiten unlöslich sind. Überraschenderweise erhält man hierbei Lösungen mit gegenüber unmodifizierten Zuckern extrem reduzierter Viskosität und verbesserter Emulgierbarkeit bzw. Mischbarkeit mit den verschiedensten nieder- und höhermolekularen Polyhydroxylverbindungen.

In den mit Dialkylphosphit modifizierten Zuckern bilden sich je nach Temperaturlage Gleichgewichte aus zwischen freiem Dialkylphosphit, Hydroxymethanphosphonsäureestern der Konstitution

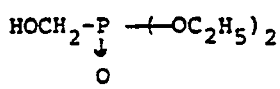

und

α-Hydroxyphosphonsäureestern der Konstitutionen

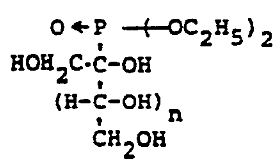

n = 2-6

Bei höheren Temperaturen (oberhalb ca. 35 °C) und insbesondere in Gegenwart von katalytischen Mengen an anorganischen Basen oder bevorzugt tertiären Aminen wie Triäthylamin oder Dimethylbenzylamin gehen diese Verbindungen Umlagerungsreaktionen und Umesterungsreaktionen unter Alkoholabspaltung ein und es entstehen cyclische Phosphite der Zucker bzw. über intermolekulare Verknüpfung von Zuckern höhermolekulare Polyphosphite bzw. Zucker-Ester der Hydroxymethylphosphonsäure. Je nach abgespaltener Alkoholmenge lassen sich beliebige Umesterungsgrade erzielen und damit z. B. Viskositäten von ca. 300 mPas bei 20 °C bis ca. 110 000 mPas bei 20 °C einstellen.

Alle eben beschriebenen gegenüber Formaldehyd reaktiven Verbindungen können aber gegebenenfalls auch im Anschluß an die erfindungsgemäße Methylolierungsreaktion zugesetzt werden, um freien Formaldehyd in den Verfahrensprodukten zu binden, wenn ein Überschuß an Formaldehyd eingesetzt worden war.

Auch die technisch interessanten Gemische aus den erfindungsgemäßen verzweigten Zuckern und den eben beschriebenen gegebenenfalls methylolierten Aminoplast und Phenoplastmonomeren, Aldehyden und Ketonen sowie Phosphiten im Gewichtsverhältnis 99 : 1 bis 5 : 95, vorzugsweise 98 : 2 bis 50 : 50, besonders bevorzugt 95 : 5 bis 70 : 3, sind Gegenstand der Erfindung, beispielsweise Gemische aus den α- bzw. α'-methylolierten Zuckern und Dimethyl- und/oder Diäthylphosphit ; Caprolactam ; N-methyloliertem Caprolactam, Pyrrolidon, Harnstoff, Melamin, Dicyandiamid, Dimethyl- und Diäthylharnstoff ; Kondensationsprodukten aus 1 Mol Anilin und 1-5 Mol Formaldehyd ; Mono-, Di- und Trimethylolphenol ; Resolen ; mono- und polymethylolierten niedermolekularen Aldehyden und Ketonen wie Cyclohexanon, Acetaldehyd, Propionaldehyd, n-Butyraldehyd, Aceton, Methyläthylketon und Methylisobutylketon.

Die erfindungsgemäßen, neuen verzweigten Zucker sind zahlreichen Reaktionen zu wertvollen technischen Zuckerderivaten zugänglich, z. B. Hydrierung zu verzweigten Alkoholen ; OH$^{\ominus}$- oder H$^{\oplus}$-katalysierte Umsetzungen (vorzugsweise mit Lewis-Säuren als Katalysator) mit Äthylenoxid, Propylenoxid oder Epichlorhydrin zu Polyäthern ; Herstellung von Polyestern ; inter- und intramolekulare Acetalbildung ; Acylierungsreaktion mit Essigsäureanhydrid, Keten oder Diketen ; Cyanäthylierung mit Acrylnitril und anschließende Hydrierungsreaktionen ; Herstellung von nichtionogenen oberflächenaktiven Verbindungen, z. B. durch Umsetzung mit Fettsäuren oder langkettigen aliphatischen Monoisocyanaten.

Eine interessante Variante des erfindungsgemäßen Verfahrens besteht darin, den Restformaldehyd durch Ansäuern auf einen pH-Wert von ca. 1-3 zu binden, wobei unter Wasserabspaltung — gegebenenfalls in Gegenwart von Borsäure als Katalysator — intra- bzw. intermolekulare Acetale gebildet werden.

Intermolekulare Acetalbildung (idealisiert) :

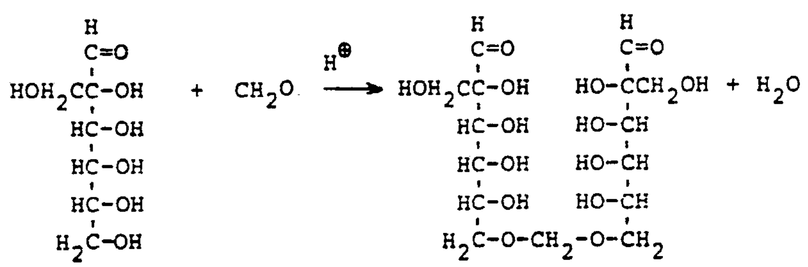

Derartige durch Acetalbildung modifizierte Endprodukte haben vorteilhafterweise geringe Viskositäten, wodurch ihre Mischbarkeit bzw: Emulgierbarkeit mit den bei der Polyurethanherstellung eingesetzten höhermolekularen Polyhydroxylverbindungen wesenclich verbessert wird.

Aus den erfindungsgemäßen verzweigten Zuckern können gegebenenfalls nach an sich bekannten Verfahren durch Reduktion in einfacher Weise mehrwertige Alkohole gewonnen werden. So gelingt z. B. die Reduktion direkt aus der erhaltenen wäßrigen Lösung schon bei Raumtemperatur mit Natriumborhydrid ; sie kann aber z. B. auch auf elektrolytischem Weg erfolgen. Auch die katalytische Hydrierung mit Wasserstoff ist möglich. Hierfür können prinzipiell alle Verfahren, die bei der Reduktion von Zuckern zu Zuckeralkoholen zum Stand der Technik gehören, angewandt werden. Besonders günstig ist die Hydrierung mit Raney-Nickel in Mengen von 5-20 Gew.-%, bezogen auf zu reduzierenden Zucker, bei Wasserstoffdrucken von 50-200 kg/cm$^2$ und Temperaturen von 20-200 °C, jedoch können mit ähnlich gutem Erfolg auch Katalysatoren, die Nickel, Kobalt, kupfer, Platin, Rhodium oder Palladium auf inerten Trägern enthalten, verwendet werden.

Die erfindungsgemäß hergestellten verzweigten Zucker sind interessante Lösungsvermittler bzw. Lösungsmittel für schwerlösliche Metallhydroxide, beispielsweise für die Hydroxide von Calcium, Barium, seltenen Erden, Strontium, Beryllium, Zink, Magnesium, Blei, Thallium, zweiwertigem Chrom, zweiwertigem Mangan, zwei- und dreiwertigem Eisen, Aluminium, zweiwertigem Zinn und zwei- und dreiwertigem Kobalt. Derartige mit Metallhydroxiden der verschiedensten Art angereicherte Zuckerlösungen stellen wertvolle Katalysatoren für die Reaktion von Isocyanaten mit Wasser bzw. Polyhydroxylverbindungen dar.

Die erfindungsgemäßen verzweigten Zucker stellen in vielen Fällen einheitliche Verbindungen dar ;

sie sind z. B. auch zur Synthese von biologischen Wirkstoffen, Flammschutzmitteln und huminsäureartigen vernetzten Hochpolymeren bislang unbekannter Konstitution geeignet.

Die erfindungsgemäß erhaltenen Verfahrensprodukte sind insbesondere als Polyolkomponente bei der Herstellung von Polyurethankunststoffen geeignet.

Gegenstand der Erfindung ist somit auch die Verwendung der oben beschriebenen Umsetzungsprodukte von $C_6$-Zuckern und ihrer Oligomeren mit einem Molekulargewicht bis zu 2 000 zur Herstellung von gegebenenfalls zellförmigen Polyurethankunststoffen durch Umsetzung von

A) Polyisocyanaten mit

B) Polyhydroxylverbindungen sowie gegebenenfalls

C) weiteren, mindestens zwei aktive Wasserstoffatome aufweisenden Verbindungen mit einem Molekulargewicht zwischen 62 und 10 000, gegebenenfalls in Anwesenheit von

D) Treibmitteln, Katalysatoren und weitern an sich bekannten Zusatzstoffen, welches dadurch gekennzeichnet ist, daß als Komponente B die erfindungsgemäßen Umsetzungsprodukte von $C_6$-Zuckern bzw. ihrer Oligomeren mit einem Molekulargewicht bis zu 2 000 eingesetzt werden, wobei gegebenenfalls ein Teil oder die Gesamtmenge dieser Umsetzungsprodukte von $C_6$-Zuckern bzw. ihrer Oligomeren zunächst hydriert und/oder oxalkyliert werden und dann als Komponente B eingesetzt werden.

Für die Herstellung von Polyurethankunststoffen werden eingesetzt :

1. Als Ausgangskomponenten aliphatische, cycloaliphatische, araliphatische, aromatische und heterocyclische Polyisocyanate, wie sie z. B. von W. Siefken in Justus Liebigs Annalen der Chemie, 562, Seiten 75 bis 136 beschrieben werden, beispielsweise solche der Formel

$$Q(NCO)_n$$

in der

n = 2-4, vorzugsweise 2,

und

Q einen aliphatischen Kohlenwasserstoffrest mit 2-18, vorzugsweise 6-10 C-Atomen,
einem cycloaliphatischen Kohlenwasserstoffrest mit 4-15, vorzugsweise 5-10 C-Atomen,
einen aromatischen Kohlenwasserstoffrest mit 6-15, vorzugsweise 6-13 C-Atomen,
oder einen araliphatischen Kohlenwasserstoffrest mit 8-15, vorzugsweise 8-13 C-Atomen,

bedeuten, z. B. Äthylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Hexahydro-1,3- und/oder 1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4' -und/oder -4,4'-diisocyanat und Naphthylen-1,5-diisocyanat.

Ferner kommen beispielsweise erfindungsgemäß in Frage : Triphenylmethan-4,4', 4''-triisocyanat, Polyphenyl-polymethylenpolyisocyanate, wie sie durch Anilin-Formaldehyd-Kondensation und anschließende Phosgenierung erhalten und z. B. in den GB-Patentschriften 874 430 und 848 671 beschrieben werden, m- und p-Isocyanatophenylsulfonyl-isocyanate gemäß der US-Patentschrift 3 454 606, perchlorierte Arylpolyisocyanate, wie sie z. B. in der DE-Auslegeschrift 1 157 601 (US-Patentschrift 3 277 138) beschrieben werden, Carbodiimidgruppen aufweisende Polyisocyanate, wie sie in der DE-Patentschrift 1 092 007 (US-Patentschrift 3 152 162) sowie in den DE-Offenlegungsschriften 2 504 400, 2 537 685 und 2 552 350 beschrieben werden, Norbornan-Diisocyanate gemäß US-Patentschrift 3 492 330, Allophanatgruppen aufweisende Polyisocyanate, wie sie z. B. in der GB-Patentschrift 994 890, der BE-Patentschrift 761 626 und der NL-Patentanmeldung 7 102 524 beschrieben werden, Isocyanuratgruppen aufweisende Polyisocyanate, wie sie z. B. in der US-Patentschrift 3 001 973, in den DE-Patentschriften 1 022 789, 1 222 067 und 1 027 394 sowie in den DE-Offenlegungsschriften 1 929 034 und 2 004 048 beschrieben werden, Urethangruppen aufweisende Polyisocyanate, wie sie z. B. in der BE-Patentschrift 752 261 oder in den US-Patentschriften 3 394 164 und 3 644 457 beschrieben werden, acylierte Harnstoffgruppen aufweisende Polyisocyanate gemäß der DE-Patentschrift 1 230 778, Biuretgruppen aufweisende Polyisocyanate, wie sie z. B. in den US-Patentschriften 3 124 605, 3 201 372 und 3 124 605 sowie in der GB-Patentschrift 889 050 beschrieben werden, durch Telomerisationsreaktionen hergestellte Polyisocyanate, wie sie z. B. in der US-Patentschrift 3 654 106 beschrieben werden, Estergruppen aufweisende Polyisocyanate, wie sie z. B. in den GB-Patentschriften 965 474 und 1 072 956, in der US-Patentschrift 3 567 763 und in der DE-Patentschrift 1 231 688 genannt werden, Umsetzungsprodukte der obengenannten Isocyanate mit Acetalen gemäß der DE-Patentschrift 1 072 385 und polymere Fettsäureester enthaltende Polyisocyanate gemäß der US-Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z. B. das 2,4- und 2,6-Toluylendiisocyanat sowie beliebige Gemische dieser Isomeren (« TDI »), Polyphenylpolymethylen-polyisocyanate, wie sie durch Anilin-Formaldehyd-kondensation und anschließende Phosgenierung hergestellt werden (« rohes MDI ») und Carbodiimidgruppen, Urethangruppen, Allophanatgruppen Isocyanuratgruppen, Harnstoffgruppen oder Biuretgruppen aufweisenden Polyisocyanate (« modifizierte Polyisocyanate »), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten.

2. Als Ausgangskomponenten ferner gegebenenfalls Verbindungen mit mindestens zwei gegenüber Isocyanaten reaktionsfähigen Wasserstoffatomen von einem Molekulargewicht in der Regel von 400-10 000. Hierunter versteht man neben Aminogruppen, Thiolgruppen oder Carboxylgruppen aufweisenden Verbindungen vorzugsweise Hydroxylgruppen aufweisende Verbindungen, insbesondere zwei bis acht Hydroxylgruppen aufweisende Verbindungen, speziell solche vom Molekulargewicht 500 bis 7 000, vorzugsweise 1 000 bis 5 000, z. B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind :

a) Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele für solche Carbonsäuren und deren Derivate seien genannt :

Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit monomeren ungesättigten Fettsäuren, wie Ölsäure ; Terephthalsäuredimethylester und Terephthalsäure-bis-glykolester. Als mehrwertige Alkohole kommen z. B. Äthylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Formit, Methylglykosid, ferner Diäthylenglykol, Triäthylenglykol, Tetraäthylenglykol und höhere Polyäthylenglykole, Dipropylenglykol und höhere Polypropylenglykole sowie Dibutylenglykol und höhere Polybutylenglykole in Frage. Die Polyester können anteilig endständige Carboxylgruppen aufweisen. Auch Polyester aus Lactonen, z. B. ε-Caprolacton, oder aus Hydroxycarbonsäuren, z. B. ω-Hydroxycapronsäure, sind einsetzbar.

b) Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z. B. durch Polymerisation von Epoxiden wie Äthylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Äthylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Äthylenglykol, Propylenglykol-(1,3) oder -(1,2), Trimethylolpropan, Glycerin, Sorbit, 4,4'-Dihydroxy-diphenylpropan, Anilin, Äthanolamin oder Äthylendiamin hergestellt. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch OH-Gruppen aufweisende Polybutadiene sind erfindungsgemäß geeignet.

c) Unter den Polythioäthern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten z. B. um Polythiomischäther, Polythioätherester oder Polythioätheresteramide.

d) Als Polyacetale kommen z. B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale wie z. B. Trioxan (DE-Offenlegungsschrift 1 694 128) lassen sich erfindungsgemäß geeignete Polyacetale herstellen.

e) Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diäthylenglykol, Triäthylenglykol, Tetraäthylenglykol oder Thiodiglykol mit Diarylcarbonaten, z. B. Diphenylcarbonat, oder Phosgen hergestellt werden können (DE-Auslegeschriften 1 694 080, 1 915 908 und 2 221 751 ; DE-Offenlegungsschrift 2 605 024).

f) Zu den Polyesteramiden und Polyamiden zählen z. B. die aus mehrwertigen gesättigten oder ungesättigten Carbonsäuren bzw. deren Anhydriden und mehrwertigen gesättigten oder ungesättigten

Aminoalkoholen, Diaminen, Polyaminen und deren Mischungen gewonnenen, vorwiegend linearen Kondensate.

g) Auch bereits Urethan- oder Harnstoffgruppen enthaltende Polyhydroxylverbindungen sowie gegebenenfalls modifizierte natürliche Polyole, wie Rizinusöl oder Kohlenhydrate, z. B. Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

h) Die genannten Polyhydroxylverbindungen können vor ihrer Verwendung im Polyisocyanat-Polyadditionsverfahren noch in der verschiedensten Weise modifiziert werden : So läßt sich gemäß DE-Offenlegungsschriften 2 210 839 (US-Patentschrift 3 849 515) und 2 544 195 ein Gemisch aus verschiedenen Polyhydroxylverbindungen (z. B. aus einem Polyäther- und einem Polyesterpolyol) durch Verätherung in Gegenwart einer starken Säure zu einem höhermolekularen Polyol kondensieren, welches aus über Ätherbrücken verbundenen verschiedenen Segmenten aufgebaut ist. Es ist auch möglich, z. B. gemäß DE-Offenlegungsschrift 2 559 372 in die Polyhydroxylverbindungen Amidgruppen oder gemäß DE-Offenlegungsschrift 2 620 487 durch Umsetzung mit polyfunktionellen Cyansäureestern Triazingruppen einzuführen. Durch Umsetzung eines Polyols mit einer weniger als äquivalenten Menge eines Diisocyanatocarbodiimids und anschließende Reaktion der Carbodiimidgruppe mit einem Amin, Amid, Phosphit oder einer Carbonsäure erhält man Guanidin-, Phosphonoformamidin-bzw. Acylharnstoffgruppen aufweisende Polyhydroxyl-verbindungen (DE-Offenlegungsschriften 2 714 289, 2 714 292 und 2 714 293). Von besonderem Interesse ist es in manchen Fällen, die höhermolekularen Polyhydroxylverbindungen durch Reaktion mit Isatosäureanhydrid vollständig oder teilweise in die entsprechenden Anthranilsäureester überzuführen, wie es in den DE-Offenlegungsschriften 2 019 432 und 2 619 840 bzw. den US-Patentschriften 3 808 250, 3 975 428 und 4 016 143 beschrieben ist. Man erhält auf diese Weise höhermolekulare Verbindungen mit endständigen aromatischen Aminogruppen.

Durch Umsetzung von NCO-Präpolymeren mit Hydroxylgruppen aufweisenden Enaminen, Aldiminen oder Ketiminen und anschließende Hydrolyse erhält man gemäß DE-Offenlegungsschrift 2 546 536 bzw. US-Patentschrift 3 865 791 höhermolekulare, endständige Aminogruppen aufweisende Verbindungen. Weitere Herstellungsverfahren für höhermolekulare Verbindungen mit endständigen Aminogruppen oder Hydrazidgruppen werden in der DE-Offenlegungsschrift 1 694 152 (US-Patentschrift 3 625 871) beschrieben.

i) Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den oben genannten, Hydroxylgruppen aufweisenden Verbindungen ablaufen läßt. Derartige Verfahren sind beispielsweise in den DE-Auslegeschriften 1 168 075 und 1 260 142, sowie den DE-Offenlegungsschriften 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-Patentschrift 3 869 413 bzw. DE-Offenlegungsschrift 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z. B. durch Polymerisation von Styrol und Acrylnitril in Gegenwart von Polyäthern (US-Patentschriften 3 383 351, 3 304 273, 3 523 093, 3 110 695 ; DE-Auslegeschrift 1 152 536) oder Polycarbonatpolyolen (DE-Patentschrift 1 769 795 ; US-Patentschrift 3 637 909) erhalten werden, sind für das erfindungsgemäße Verfahren geeignet. Bei Verwendung von Polyätherpolyolen, welche gemäß den DE-Offenlegungsschriften 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-Offenlegungsschriften 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Vertreter der genannten erfindungsgemäß zu verwendenden Verbindungen sind z. B. in High Polymers, Vol. XVI, « Polyurethanes, Chemistry and Technology », verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z. B. auf den Seiten 45-71, beschrieben. Selbstverständlich können Mischungen der obengenannten Verbindungen mit mindestens zwei gegenüber Isocyanaten rekationsfähigen Wasserstoffatomen mit einem Molekulargewicht von 400-10 000, z. B. Mischungen von Polyäthern und Polyestern, eingesetzt werden.

Von besonderem Vorteil ist es dabei in manchen Fällen, niedrigschmelzende und hochschmelzende Polyhydroxylverbindungen miteinander zu kombinieren (DE-Offenlegungsschrift 2 706 297).

3. Gegebenenfalls als Ausgangskomponenten Verbindungen mit mindestens zwei gegenüber Isocyanaten reaktionsfähigen Wasserstoffatomen und einem Molekulargewicht von 32 bis 400. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf.

Auf in diesem Fall können Mischungen von verschiedenen Verbindungen mit mindestens zwei gegenüber Isocyanaten reaktionsfähigen Wasserstoffatomen mit einem Molekulargewicht von 62 bis 400 verwendet werden.

Als Beispiele für derartige Verbindungen seien genannt :

Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol (US-Patentschrift 3 723 392), Glyzerin, Trimethylolpropan, Hexantriol-(1,2,6), Trimethyloläthan, Pentaerythrit, Chinit, Mannit und Sorbit, Ricinusöl, Diäthylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4'-Dihydroxy-diphenylpropan, Di-hydroxymethyl-hydrochinon, Äthanolamin, Diäthanolamin, N-Methyldiäthanolamin, Triäthanolamin und 3-Aminopropanol.

Als Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht (DE-Offenlegungsschrift 2 638 759).

Erfindungsgemaß geeignete aliphatische Diamine sind beispielsweise Äthylendiamin, 1,4-Tetramethylendiamin, 1,11-Undecamethylendiamin, 1,12-Dodecamethylendiamin sowie deren Gemische, 1-Amino-3,3,5-trimethyl-5-aminomethylcyclohexan (« Isophorondiamin »), 2,4- und 2,6-Hexahydrotoluylendiamin sowie deren Gemische Perhydro-2,4'- und 4,4'-diaminodiphenylmethan, p-Xylylendiamin, Bis-(3-aminopropyl)-methylamin, Diamino-perhydroanthrazene (DE-Offenlegungsschrift 2 638 731) und cycloaliphatische Triamine gemäß DE-Offenlegungsschrift 2 614 244. Auch Hydrazin und substituierte Hydrazine, z. B. Methylhydrazin, N,N'-Dimethylhydrazin und deren Homologe sowie Säuredihydrazide kommen erfindungsgemäß in Betracht, z. B. Carbodihydrazid, Oxalsäuredihydrazid, die Dihydrazide von Malonsäure, Bernsteinsäure, Glutarsäure, Adipinsäure, β-Methyladipinsäure, Sebazinsäure, Hydracrylsäure und Terephthalsäure ; Semicarbazido-alkylen-hydrazide wie z. B. β-Semicarbazidopropionsäurehydrazid (DE-Offenlegungsschrift 1 770 591), Semicarbazido-alkylencarbazinester wie z. B. 2-Semicarbazidoäthyl-carbazinester (DE-Offenlegungsschrift 1 918 504) oder auch Amino-semicarbazid-Verbindungen wie z. B. β-Aminoäthyl-semicarbazido-carbonat (DE-Offenlegungsschrift 1 902 931). Zur Steuerung ihrer Reaktivität können die Aminogruppen ganz oder teilweise durch Aldimin-bzw. Ketimin-Gruppen blockiert sein (US-Patentschrift 3 734 894 ; DE-Offenlegungsschrift 2 637 115).

Als Beispiele für aromatische Diamine seien Bisanthranilsäureester gemäß den DE-Offenlegungsschriften 2 040 644 und 2 160 590, 3,5- und 2,4-Diaminobenzoesäureester gemäß DE-Offenlegungsschrift 2 025 900, die in den DE-Offenlegungsschriften 1 803 635 (US-Patentschriften 3 681 290 und 3 736 350), 2 040 650 und 2 160 589 beschriebenen estergruppenhaltigen Diamine, die Äthergruppen aufweisenden Diamine gemäß DE-Offenlegungsschriften 1 770 525 und 1 809 172 (US-Patentschriften 3 654 364 und 3 736 295), gegebenenfalls in 5-Stellung substituierte 2-Halogen-1,3-Phenylendiamine (DE-Offenlegungsschriften 2 001 772, 2 025 896 und 2 065 869), 3,3'-Dichlor-4,4'-diamino-diphenylmethan, Toluylendiamin, 4,4'-Diaminodiphenylmethan, 4,4'-Diaminodiphenyldisulfide (DE-Offenlegungsschrift 2 404 976), Diaminodiphenyldithioäther (DE-Offenlegungsschrift 2 509 404), durch Alkylthiogruppen substituierte aromatische Diamine (DE-Offenlegungsschrift 2 638 760), Diaminobenzolphosphonsäureester (DE-Offenlegungsschrift 2 459 491), Sulfonat- oder Carboxylatgruppen enthaltende aromatische Diamine (DE-Offenlegungsschrift 2 720 166) sowie die in der DE-Offenlegungsschrift 2 635 400 aufgeführten hochschmelzenden Diamine genannt. Beispiele für aliphatisch-aromatische Diamine sind die Aminoalkylthioaniline gemäß DE-Offenlegungsschrift 2 734 574.

Als Kettenverlängerungsmittel können erfindungsgemäß auch Verbindungen wie 1-Mercapto-3-aminopropan, gegebenenfalls substituierte Aminosäuren, z. B. Glycin, Alanin, Valin, Serin und Lysin sowie gegebenenfalls substituierte Dicarbonsäuren, beispielsweise Bernsteinsäure, Adipinsäure, Phthalsäure, 4-Hydroxyphthalsäure und 4-Aminophthalsäure verwendet werden.

Ferner können gegenüber Isocyanaten monofunktionelle Verbindungen in Anteilen von 0,01 bis 10 Gew.-%, bezogen auf Polyurethanfeststoff, als sogenannte Kettenabbrecher mitverwendet werden. Derartige monofunktionelle Verbindungen sind z. B. Monoamine wie Butyl- und Dibutylamin, Octylamin, Stearylamin, N-Methylstearylamin, Pyrrolidin, Piperidin und Cyclohexylamin, Monoalkohole wie Butanol, 2-Äthylhexanol, Octanol, Dodecanol, die verschiedenen Amylalkohole, Cyclohexanol, Äthylenglykolmonoäthyläther.

4. Gegebenenfalls als Hilfs- und Zusatzmittel :

a) Wasser und/oder leicht flüchtige anorganische oder organische Substanzen als Treibmittel. Als organische Treibmittel kommen z. B. Aceton, Äthylacetat, halogensubstituierte Alkane wie Methylenchlorid, Chloroform, Äthylidenchlorid, Vinylidenchlorid, Monofluortrichlormethan, Chlordifluormethan, Dichlordifluormethan, ferner Butan, Hexan, Heptan oder Diäthyläther, als anorganische Treibmittel z. B. Luft, $CO_2$ oder $N_2O$, in Frage. Eine Treibwirkung kann auch durch Zusatz von bei Temperaturen über Raumtemperatur unter Abspaltung von Gasen, beispielsweise von Stickstoff, sich zersetzenden Verbindungen, z. B. Azoverbindungen wie Azodicarbonamid oder Azoisobuttersäurenitril, erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z. B. auf den Seiten 108 und 109, 453 bis 455 und 507 bis 510 beschrieben.

b) Katalysatoren der an sich bekannten Art, z. B. tertiäre Amine, wie Triäthylamin, Tributylamin, N-Methyl-morpholin, N-Äthyl-morpholin, N,N,N',N'-Tetramethyl-äthylendiamin, Pentamethyl-diäthylentriamin und höhere Homologue (DE-Offenlegungsschriften 2 624 527 und 2 624 528), 1,4-Diazabicyclo-(2,2,2)-octan, N-Methyl-N'-dimethylaminoäthylpiperazin, Bis-(dimethylaminoalkyl)-piperazine (DE-Offenlegungsschrift 2 636 787), N,N-Dimethylbenzylamin, N,N-Dimethylcyclohexylamin, N,N-Diäthylbenzylamin, Bis-(N,N-diäthylaminoäthyl)-adipat, N,N,N',N'-Tetramethyl-1,3-butandiamin, N,N-Dimethyl-β-phenyläthylamin, 1,2-Dimethylimidazol, 2-Methylimidazol, monocyclische und bicyclische Amidine (DE-Offenlegungsschrift 1 720 633), Bis-(dialkylamino)alkyl-äther (US-Patentschrift 3 330 782, DE-Auslegeschrift 1 030 558, DE-Offenlegungsschriften 1 804 361 und 2 618 280) sowie Amidgruppen (vorzugsweise Formamidgruppen) aufweisende tertiäre Amine gemäß den DE-Offenlegungsschriften 2 523 633 und 2 732 292). Als Katalysatoren kommen auch an sich bekannte Mannichbasen aus sekundären Aminen, wie Dimethylamin, und Aldehyden, vorzugsweise Formaldehyd, oder Ketonen wie Aceton, Methyläthylketon oder Cyclohexanon und Phenolen, wie Phenol, Nonylphenol oder Bisphenol, in Frage.

Gegenüber Isocyanatgruppen aktive Wasserstoffatome aufweisende tertiäre Amine als Katalysator sind z. B. Triäthanolamin, Triisopropanolamin, N-Methyl-diäthanolamin, N-Äthyl-diäthanolamin, N,N-Dimethyläthanolamin, deren Umsetzungsprodukte mit Alkylenoxiden wie Propylenoxid und/oder Äthylenoxid sowie sekundär-tertiäre Amine gemäß DE-Offenlegungsschrift 2 732 292.

Als Katalysatoren kommen ferner Silaamine mit Kohlenstoff-Silizium-Bindungen, wie sie z. B. in der DE-Patentschrift 1 229 290 (entsprechend der US-Patentschrift 3 620 984) beschrieben sind, in Frage, z. B. 2,2,4-Trimethyl-2-silamorpholin und 1,3-Diäthylaminomethyl-tetramethyl-disiloxan.

Als Katalysatoren kommen auch stickstoffhaltige Basen wie Tetraalkylammoniumhydroxide, ferner Alkalihydroxide wie Natriumhydroxid, Alkaliphenolate wie Natriumphenolat oder Alkalialkoholate wie Natriummethylat in Betracht. Auch Hexahydrotriazine können als Katalysatoren eingesetzt werden (DE-Offenlegungsschrift 1 769 043).

Die Reaktion zwischen NCO-Gruppen und Zerewitinoffaktiven Wasserstoffatomen wird auch durch Lactame und Azalactame stark beschleunigt, wobei sich zunächst ein Assoziat zwischen dem Lactam und der Verbindung mit acidem Wasserstoff ausbildet. Derartige Assoziate und ihre katalytische Wirkung werden in den DE-Offenlegungsschriften 2 062 288, 2 062 289, 2 117 576 (US-Patentschrift 3 758 444), 2 129 198, 2 330 175 und 2 330 211 beschrieben.

Erfindungsgemäß können auch organische Metallverbindungen, insbesondere organische Zinnverbindungen, als Katalysatoren, verwendet werden. Als organische Zinnverbindungen kommen neben schwefelhaltigen Verbindungen wie Di-n-octyl-zinn-mercaptid (DE-Auslegeschrift 1 769 367 ; US-Patentschrift 3 645 927) vorzugsweise Zinn(II)-salze von Carbonsäuren wie Zinn(II)-acetat, Zinn(II)-octoat, Zinn(II)-äthylhexoat und Zinn(II)-laurat und die Zinn(IV)-Verbindungen, z. B. Dibutylzinnoxid, Dibutylzinndichlorid, Dibutylzinndiacetat, Dibutylzinndilaurat, Dibutylzinnmaleat oder Dioctylzinndiacetat in Betracht.

Selbstverständlich können alle obengenannten Katalysatoren als Gemische eingesetzt werden. Von besonderem Interesse sind dabei Kombinationen aus organischen Metallverbindungen und Amidinen, Aminopyridinen oder Hydrazinopyridinen (DE-Offenlegungsschriften 2 434 185, 2 601 082 und 2 603 834).

Weitere Vertreter von erfindungsgemäß zu verwendenden Katalysatoren sowie Einzelheiten über die Wirkungsweise der Katalysatoren sind im Kunststoff-Handbuch, Band VII, herausgegeben von Vieweg und Höchtlen, Carl-Hanser-Verlag, München 1966, z. B. auf den Seiten 96 bis 102 beschrieben.

Die Katalysatoren werden in der Regel in einer Menge zwischen etwa 0,001 und 10 Gew.-%, bezogen auf die Gesamtmenge an Verbindungen mit mindestens zwei gegenüber Isocyanaten reaktionsfähigen Wasserstoffatomen, eingesetzt.

c) Oberflächenaktive Zusatzstoffe, wie Emulgatoren und Schaumstabilisatoren. Als Emulgatoren kommen z. B. die Natriumsalze von Ricinusöl-sulfonaten oder Salze von Fettsäuren mit Aminen wie ölsaures Diäthylamin oder stearinsaures Diäthanolamin infrage. Auch Alkali- oder Ammoniumsalze von Sulfonsäuren wie etwa von Dodecylbenzolsulfonsäure oder Dinaphtyl-methandisulfonsäure oder von Fettsäuren wie Ricinolsäure oder von polymeren Fettsäuren können als oberflächenaktive Zusatzstoffe mitverwendet werden.

Als Schaumstabilisatoren kommen vor allem Polyäthersiloxane, speziell wasserlösliche Vertreter, infrage. Diese Verbindungen sind im allgemeinen so aufgebaut, daß ein Copolymerisat aus Äthylenoxid und Propylenoxid mit einem Polydimethylsiloxanrest verbunden ist. Derartige Schaumstabilisatoren sind z. B. in den US-Patentschriften 2 834 748, 2 917 480 und 3 629 308 beschrieben. Von besonderem Interesse sind vielfach über Allophanatgruppen verzweigte Polysiloxan-Polyoxyalkylen-Copolymere gemäß DE-Offenlegungsschrift 2 558 523.

d) Reaktionsverzögerer, z. B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und Flammschutzmittel der an sich bekannten Art, z. B. Trischloräthylphosphat, Trikresylphosphat oder Ammoniumphosphat und -polyphosphat, ferner Stabilisatoren gegen Alterungs- und Witterungseinflüsse; Weichmacher und fungistatisch und bakteriostatisch wirkende Substanzen sowie Füllstoffe wie Bariumsulfat, Kieselgur, Ruß oder Schlämmkreide.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z. B. auf den Seiten 103 bis 113 beschrieben.

Durchführung des Verfahrens zur Herstellung von Polyurethankunststoffen bei erfindungsgemäßer Verwendung der oben beschriebenen α-methylolierten $C_6$-Zucker : Die Reaktionskomponenten werden nach dem an sich bekannten Einstufenverfahren, dem Prepolymerverfahren oder dem Semiprepolymerverfahren zur Umsetzung gebracht, wobei man sich oft maschineller Einrichtungen bedient, z. B. solcher, die in der US-Patentschrift 2 764 565 beschrieben werden. Einzelheiten über Verarbeitungseinrichtungen, die auch erfindungsgemäß infrage kommen, werden im Kunststoff-Handbuch, Band VII, herausgegeben von Vieweg und Höchtlen, Carl-Hanser-Verlag, München 1966, z. B. auf den Seiten 121 bis 205 beschrieben.

Bei der Schaumstoffherstellung kann die Verschäumung auch in geschlossenen Formen durchgeführt werden. Dabei wird das Reaktionsgemisch in eine Form eingetragen. Als Formmaterial kommt Metall, z. B. Aluminium, oder Kunststoff, z. B. Epoxidharz, infrage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Man kann in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter « overcharging » gearbeitet ; eine derartige Verfahrensweise ist z. B. aus den US-Patentschriften 3 178 490 und 3 182 104 bekannt.

Bei der Formverschäumung werden vielfach an sich bekannte « äußere Trennmittel », wie Siliconöle, mitverwendet. Man kann aber auch sogenannte « innere Trennmittel », gegebenenfalls im Gemisch mit äußeren Trennmitteln, verwenden, wie sie z. B. aus den DE-Offenlegungsschriften 2 121 670 und 2 307 589 bekanntgeworden sind.

Erfindungsgemäß lassen sich auch kalthärtende Schaumstoffe herstellen (vgl. GB-Patentschrift 1 162 517, DE-Offenlegungsschrift 2 153 086).

Selbstverständlich können aber auch Schaumstoffe durch Blockverschäumung oder nach dem an sich bekannten Doppeltransportbandverfahren hergestellt werden.

Die Umsetzung der nach anschließender Hydrierung der Aldo- und Ketofunktionen erfindungsgemäß zugänglichen Polyhydroxylverbindungen (ohne Mitverwendung anderer gegenüber Isocyanaten reaktiver Komponenten) mit stark elastifizierenden Polyisocyanaten, wie z. B. Polyisocyanaten mit Biuretstruktur (DAS 1 543 178) führt zu harten, lichtechten, kratz- und lösungsmittelfesten Beschichtungen und Lacken.

Durch Propoxylierung und/oder Oxyäthylierung der erfindungsgemäßen Zucker und ihrer Hydrierungsprodukte lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialen für hochelastische Polyurethanschaumstoffe Verwendung finden.

Durch Umsetzung der erfindungsgemäß hergestellten Verfahrensprodukte und ihrer Hydrierungsprodukte mit Carbonsäuren der obengenannten Art, z. B. Phthalsäure, Isophthalsäure, Terephthalsäure, Tetra- und Hexahydrophthalsäure, Adipinsäure oder Maleinsäure nach den üblichen Verfahren der Polyesterkondensation, wie sie beispielsweise in Houben-Weyl, Methoden der organischen Chemie Band XIV/12, S. 40 beschrieben sind, lassen sich stark verzweigte Polyester synthetisieren, die als Zusätze zu Alkydharzen deren Härte verbessern. Insbesondere die Hydroxylgruppen enthaltenden Polyester, die bevorzugt aus den hydrierten erfindungsgemäßen Zuckern synthetisiert werden, sind ebenfalls als Ausgangskomponente zur Herstellung von Polyurethankunststoffen brauchbar.

Die erfindungsgemäß hergestellten Verfahrensprodukte und ihre Hydrierungsprodukte lassen sich auch sehr leicht mit langkettigen, aliphatischen Monocarbonsäuren, wie Capryl-, Caprin-, Laurin-, Myristin-, Palmitin-, Stearin-, Öl-, Linol-, Arachidon-, oder Behensäure, sowie deren Derivaten, wie z. B. den Methyl- oder Äthylestern oder auch den Anhydriden bzw. gemischten Anhydriden zu hydroxylgruppenhaltigen Estern umsetzen. Diese stellen ebenso wie Oxäthylierungsprodukte oder auch Umsetzungsprodukte der erfindungsgemäßen verzweigten Zucker und ihrer Hydrierungsprodukte mit langkettigen Monoisocyanaten, wie n-Octyl-, n-Decyl-, n-Dodecyl-, Myristyl-, Cetyl- oder Stearylisocyanat zu Carbamidsäureestern (siehe z. B. K. Lindner, Tenside Bd. III, Wissenschaftliche Verlagsgesellschaft Stuttgart, 1964, S. 2336) nichtionogene, oberflächenaktive Verbindungen dar, die als wertvolle Emulgatoren, Netzmittel oder Weichmacher Verwendung finden können.

Die erfindungsgemäßen verzweigten Zucker und ihre Hydrierungsprodukte kassen sich auch als Feuchthaltemittel in Kosmetika und Kunststoffen verwenden. Sie können aber z. B. auch als Gefrierschutzmittel dienen.

Ebenso ist ihr Einsatz als kohlenhydrathaltiges Substrat in Nährböden von Mikroorganismen möglich. Hierzu haben sich besonders diejenigen Verfahrensprodukte bewährt, die hauptsächlich aus 5 bis 9 Kohlenstoffatome enthaltenden verzweigten Monosacchariden (verzweigte Aldo- bzw. Ketozucker) bestehen.

Die folgenden Beispiele erläutern das erfindungsgemäße Verfahren. Wenn nicht anders vermerkt, sind Zahlenwerte als Gewichtsteile bzw. Gewichtsprozente zu verstehen.

## Beispiel 1

Dieses Beispiel zeigt die Umwandlung von D-Glucose in einen verzweigten, 2 primäre Hydroxylgruppen enthaltenden, bislang unbekannten $C_7$-Zucker, der eine tertiäre Hydroxylgruppe besitzt.

### Variante A

1 188 Teile Glucose. $1H_2O$ (= 6 Mol Traubenzuckerhydrat) werden in 1812 Teilen Wasser bei 40 °C gelöst. Anschließend werden 710 Teile (7,1 Mol) einer 30 %igen wäßrigen Formaldehydlösung (im folgenden « Formalinlösung » genannt) in einem Guß zugegeben. Die klare Lösung wird unter intensivem Rühren auf 80 °C aufgeheizt. Hierauf werden 36 Teile N,N-Dimethylcyclohexylamin (0,283 Mol) zugegeben und ein pH-Wert von 9,5 eingestellt, der im Verlaufe der Formaldehyd-Addition innerhalb von 40 Minuten auf 7,8 abfällt. Nach diesen 40 Minuten Reaktionszeit sind, wie eine analytische Bestimmung des freien Formaldehyds zeigt, bereits 79,3 Gew.-% der eingesetzten D-Glucose α-C-methyloliert worden. Anschließend wird weitere 40 Minuten bei 84 °C nachgerührt, bis der freie Formaldehydgehalt der Lösung auf 0,87 % abgefallen ist und ein pH-Wert von 6,4 erreicht ist ; die α-C-Methylolierung ist zu diesem Zeitpunkt zu 94 % der Theorie beendet. Die erhaltene Lösung ist lediglich schwach gelb gefärbt. Man klärt mit 10 Teilen Aktivkohle und erhält nach der Wasserentfernung bei 15 Torr im Rotationsverdampfer 1 419 Teile eines verzweigten $C_7$-Rohzuckers mit 7,6 Gew.-% Wassergehalt, welcher noch ca. 3,46 Gew.-% an Dimethylcyclohexylammoniumformiat enthält und der bei 70 °C vorteilhafterweise eine Viskosität von nur 4873 m.Pa.s besitzt, während D-Glucose bei dieser Temperatur noch kristallin ist und selbst als Glucosemonohydrat (mit ca. 9,2 Gew.-% Wassergehalt) nicht schmilzt. Durch Entsalzung an einem sauren und anschließend an einem basischen Ionenaustauscher wird der α-methylolierte, verzweigte $C_7$-Zucker vom Katalysator und Ameisensäure befreit.

Gefundenes Carbonyläquivalent : 0,45, berechnet auf wasserfreies Verfahrensprodukt.

Der erhaltene verzweigte Zucker neigt bei 7,6 % Wassergehalt nicht zur Kristallisation. Seine optische Aktivität beträgt $[\alpha_D] = +31°$.

Man acetyliert das nichtentsalzte, 7,5 % Wasser enthaltende Verfahrensprodukt mit überschüssigem Essigsäureanhydrid und 1 Gew.-% Natriumacetat als Katalysator bei 70 °C, indem man in die vorgelegte Essigsäureanhydrid-Mischung langsam das in einem Tropftrichter auf 70 °C erwärmte Verfahrensprodukt im Verlaufe von 2 Stunden eintropft. Mit zunehmendem Acetylierungsgrad geht das Verfahrensprodukt exotherm in Lösung. Anschließend werden Essigsäure und überschüssiges Essigsäureanhydrid zunächst bei 15 Torr und anschließend bei 0,2 Torr bei einer Innentemperatur von 50 °C entfernt. Das so hergestellte Hexaacetat des $C_7$-Zuckers ist in Chloroform bzw. Toluol oder Aceton löslich. Beim Aufnehmen des flüssigem Reaktionsproduktes in der gleichen Gewichtsmenge Aceton fällt das Natriumacetat aus. Man destilliert das Aceton im Vakuum ab, digeriert den wasserunlöslichen Zuckersirup 4 × mit je 500 Teilen Wasser zur Entfernung von kleinen Mengen Essigsäure und erhält hierdurch ein ausgezeichnet kristallisierendes Hexaacetat der α-C-methylolierten Glucose, das bei 89 °C schmilzt.

Gefundenes Molekulargewicht in Toluol : 469

Berechnetes Molekulargewicht : 462

Phenylhydrazon des $C_7$-Zuckers : Fp. : 198 °C.

### Variante B (bevorzugt)

Man verfährt wie unter Variante A beschrieben, verwendet ebenfalls Dimethylcyclohexylamin als Katalysator, tropft aber den Katalysator im Verlaufe von 2 Stunden in die vorlegte Mischung so zu, daß der pH-Wert der Lösung etwa zwischen 8,3 und 8,4 konstant bleibt. Hierdurch wird die α-methylolierte Glucose in 96 %iger Ausbeute und maximaler Reinheit erhalten ; sie ist von einer hervorragenden farblichen Qualität und besitzt ein Carbonyläquivalent von 0,46, berechnet auf wasserfreies Verfahrensprodukt. Fp. des Hexaacetats : 89 °C ; löslich in Chloroform und Toluol.

Variante C (bevorzugt für hohe Feststoffgehalte)

Man verfährt wie bei Variante B beschrieben, verzichtet aber auf zusätzliches Wasser als Verdünnungsmittel und löst 594 Teile (3 Mol) kristallisiertes Glucose-Hydrat bei 55 °C in 360 Teilen 30 %iger Formalinlösung (3,6 Mol), d. h. man arbeitet bei einer wesentlich höheren Konzentration der Reaktionspartner (etwa 68 %, wenn das durch Glucosehydrat eingeführte Wasser mitberücksichtigt wird). Anschließend erhöht man die Reaktionstemperatur auf 78-80 °C und leitet die exotherm verlaufende α-Additionsreaktion durch gleichmäßige Dosierung des Dimethylcyclohexylamins oder einer 30 %igen NaOH-Lösung zur pH-Steuerung zwischen 8,4-8,3, gegebenenfalls unter leichter Kühlung ein. Die α-Addition zur α-methylolierten Glucose ist bei dieser Variante bereits nach einer knappen Stunde, bei einem Formaldehyd-Endwert der Lösung von ca. 0,83 % beendet. Die erhaltene, ca. 69,3 %ige Lösung an α-C-methylolierter Glucose ist lediglich hellgelb gefärbt.

Carbonyläquivalent des entsalzten Verfahrensproduktes : 0,455, bezogen auf wasserfreies Produkt.

Ausbeute : 678 Teile (Wassergehalt 9,2 Gew.-%).

Die Viskosität einer 68 %igen wäßrigen Lösung beträgt bei 20 °C lediglich 339 m.Pa.s.

Die Viskosität des auf 9,2 % Wasser eingeengten Zuckersirups beträgt bei 50 °C 8 799 m.Pa.s

$\eta$ 60 °C = 2 905 m.Pa.s

$\eta$ 70 °C = 1 170 m.Pa.s

## Beispiel 2

Man verfährt wie in Beispiel 1, Variante B, beschrieben, verwendet jedoch nur 6 Mol an Formaldehyd und unterbricht die Reaktion bei einer Konzentration von 0,8 Gew.-%. Man erhält nach der Acetylierung des Verfahrensproduktes ein Gemisch, das aus ca. 5 Mol der hexaacetylierten α-aldolisierten Glucose und einem Mol von Pentaacetylglucose besteht.

Fp. des Gemisches : 81 °C.

Das nichtacetylierte Verfahrensprodukt ist günstigerweise flüssig, kann dadurch leichter propoxyliert, oxäthyliert, mit Epichlorhydrin oder mit Acetanhydrid umgesetzt werden. Selbst nach 6 monatiger Lagerzeit kristallisiert die D-Glucose aus dem Verfahrensprodukt nicht aus.

## Beispiel 3

Man verfährt genau wie in Beispiel 1, Variante B, beschrieben und ersetzt lediglich die 460 Teile Wasser durch 460 Teile Äthylenglykol. Nach der Wasserentfernung im Dünnschichtverdampfer bei 16 Torr erhält man eine sehr niedrigviskose Lösung des Verfahrensproduktes. Die Mischung enthält ca. 26,6 Gew.-% Äthylenglykol und besitzt bei 25 °C lediglich eine Viskosität von 3 800 m.Pa.s.

## Beispiel 4

Man verfährt wie in Beispiel 1, Variante C, beschrieben, und zwar mit 0,33 Mol 30 %iger NaOH als Katalysator, verwendet aber bei der Umsetzung anstatt 7 Mol Formaldehyd 37 Mol Formaldehyd in Form einer 37 %igen Formalinlösung (3 000 Teile).

Durch Titration verfolgt man die Formaldehydabnahme. Nach 3 Stunden sind etwa 6 Mol an Formaldehyd verbraucht. Die erhaltene Lösung wird nun bei 0,4 Torr im Dünnschichtverdampfer eingeengt und man erhält das idealisierte Poly-Halbacetal des $C_7$-Zuckers :

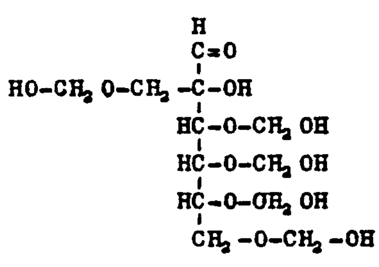

Ausbeute : 2 350 g (bei einem Gehalt von 8 Gew.-% Wasser) ; Durchschnittsmolekulargewicht : 360.

Durch Modellversuche wurde gefunden, daß die tert. Hydroxylgruppe am $C_2$-Atom unter den angegebenen Bedingungen keine Halbacetalbildung eingeht.

## Beispiel 5

Man verfährt nach Beispiel 1, Verfahrensvariante B, verwendet aber als Formaldehydquelle ein Formaldehyd-Synthesegas, wie es bei der großtechnischen Produktion von Formaldehyd anfällt und das etwa folgende Zusammensetzung aufweist :

| | $Nm^3/h$ | Vol.% |
|---|---|---|
| $N_2$ | 6,73 | 31,607 |
| $H_2$ | 1,35 | 6,441 |
| $CO_2$ | 0,31 | 1,477 |
| $CO$ | 0,02 | 0,099 |
| $\overset{\overset{O}{\Vert}}{HC}\text{-}OCH_3$ | 0,01 | 0,066 |
| $CH_4$ | 0,01 | 0,066 |
| $CH_2O$ | 3,83 | 17,565 |
| $H_2O$ | 8,93 | 42,012 |
| $CH_3OH$ | 0,14 | 0,667 |
| | 21,33 $Nm^3/h$ | 100,000 Vol.% |

Man führt die Reaktion mit dem Synthesegas diskontinuierlich bei 80-85 °C durch : 2,1 Stunden lang werden pro Stunde 426 l des Synthesegases, enthaltend etwa 102 g Formaldehyd, in 6 Mol der wäßrigen Glucose-Lösung des Beispiels 1 unter Rühren eingeleitet (insgesamt ca. 210 g = 7 mol absorbierter Formaldehyd). Die Prozeßgase werden über ein Einleitungsrohr, das 0,5 cm über dem Boden eines zylindrischen Reaktionsgefäßes endet, in die Absorptionsflüssigkeit eingeleitet. Obwohl die Absorptionsflüssigkeit nicht im Gegenstrom zum Prozeßgas umgepumpt wird und keine Füllkörper vorhanden sind, d. h. apparativ ungünstigere Verhältnisse vorliegen als bei kontinuierlicher Verfahrensweise, wird der Formaldehyd aus dem Prozeßgas zu etwa 98 % absorbiert und in α-C-methylolierte Glucose umgewandelt.

Carbonyläquivalent der erhaltenen α-C-methylolierten Glucose : 0,465

Beispiel 6

Man verfährt wie in Beispiel 1, Variante B, beschrieben, verwendet aber andere basische organische bzw. anorganische Katalysatoren in jeweils identischer molarer Menge :

a) 0,28 Mol einer wäßrigen 30 %igen Trimethylaminlösung
b) 0,28 Mol einer wäßrigen 50 %igen Triäthylaminlösung
c) 0,28 Mol einer wäßrigen 50 %igen Endoäthylenpiperazinlösung
d) 0,28 Mol einer 50 %igen wäßrigen Lösung des bicyclischen Amidins

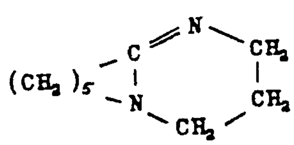

e) 0,28 Mol Cholin $[(CH_3)_3N—CH_2—CH_2OH]^{\oplus}$ $OH^{\ominus}$ in ca. 8 %iger methanolischer Lösung
f) 0,28 Mol einer 30 %igen NaOH-Lösung
g) 0,28 Mol Natriumcyanid
h) 0,28 Mol Kaliumcyanid

Aufarbeitung und Reinigung der verzweigten $C_7$-Zucker erfolgt wie in Beispiel 1 beschrieben.

Bei den Versuchen a) bis h) werden folgende Carbonyläquivalente, bezogen auf 100 g wasserfreies Verfahrensprodukt, gefunden :

a) Carbonyläquivalent : 0,45
b) Carbonyläquivalent : 0,44
c) Carbonyläquivalent : 0,45
d) Carbonyläquivalent : 0,46
e) Carbonyläquivalent : 0,46
f) Carbonyläquivalent : 0,45
g) Carbonyläquivalent : 0,468
h) Carbonyläquivalent : 0,466

Das theoretische Carbonyläquivalent der α-methylolierten D-Glucose beträgt : 0,476

Die vollständige Acetylierung gemäß Beispiel 1 ergibt bei allen Proben in ca. 96 %iger Ausbeute das ausgezeichnete kristallisierende neue Hexaacetyl-Derivat, das bei 89 °C schmilzt. Die Umsetzung mit Phenylhydrazin liefert kein Osazon sondern das gut kristallisierende gelbe Hydrazon vom Fp. 198 °C der Konstitution :

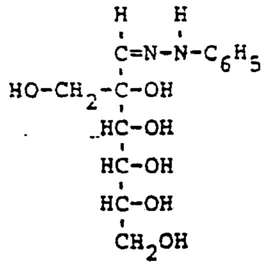

Die Tatsache, daß die erfindungsgemäßen $C_7$-Zucker mit Phenylhydrazin keine Osazone bilden, ist ein eindeutiger Konstitutionsbeweis für die α-Addition des Formaldehyds am C-Atom 2 der Glucose.

Beispiel 7

Man verfährt wie in Beispiel 1, Variante B, beschrieben, verwendet aber als Katalysatoren

a) 0,14 Mol

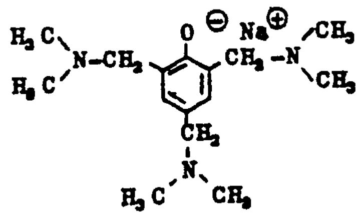

b) 0,14 Mol 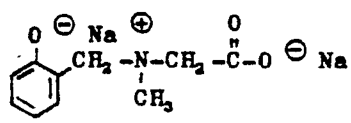

c) 0,14 Mol 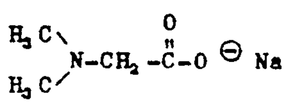

d) 0,14 Mol 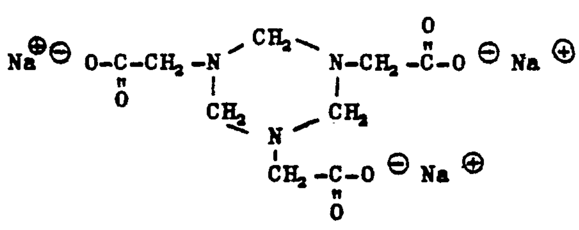

Bei den Versuchen a) bis d) werden folgende Carbonyläquivalente, bezogen auf 100 g wasserfreies Verfahrensprodukt, gefunden :

a) Carbonyläquivalent : 0,45
b) Carbonyläquivalent : 0,46
c) Carbonyläquivalent : 0,45
d) Carbonyläquivalent : 0,46

Beispiel 8

Man verfährt nach Beispiel 1, Variante B, führt aber die α-Additionsreaktion des Formaldehyds an D-Glucose mit 30 %iger Natronlauge als Katalysator und statt bei pH = 8,4-8,5 etwa bei pH = 7,5 aus. Reaktionstemperatur : 82 °C.

Die Reaktionsdauer der α-Addition beträgt bei pH = 7,5 bis zu einem Restgehalt von 0,8 % Formaldehyd 11 Stunden und 40 Minuten.

Die Aufarbeitung erfolgt wie in Beispiel 1. beschrieben. Carbonyläquivalent, bezogen auf 100 g wasserfreies Verfahrensprodukt : 0,46.

Durch den um ca. 1 Einheit verminderten pH-Wert wird also die Reaktionszeit gegenüber der bevorzugten pH-Führung bei 8,4-8,5 größenordnungsmäßig um den Faktor 8 verlangsamt.

Beispiel 9

Man verfährt wie in Beispiel 1, Variante C, beschrieben, verkleinert jedoch den Ansatz und setzt lediglich 2 Mol D-Glucose, 120 Teile einer 70 °C heißen, 60 %igen wäßrigen Formaldehydlösung (2,4 Mol Formaldehyd) ein und verwendet 12 Teile Dimethylcyclohexylamin als Katalysator.

Man erhält auf diese Weise ein Verfahrensprodukt mit extrem hoher Konzentration des $C_7$-Zuckers. Bei einem Wassergehalt von etwa 16,5 % besitzt das noch Dimethylcyclohexylammoniumformiat enthaltende Rohprodukt bei 25 °C lediglich eine Viskosität von 28 300 m.Pa.s.

Beispiel 10

Man verfährt nach Beispiel 1, Variante B, jedoch mit dem Unterschied, daß man die dort genannte Menge von Glucose in wäßriger Lösung zunächst nicht mit Formaldehyd versetzt, sondern 1 Stunde bei 85 °C mit 0,2 Mol Pyridin bei pH = 7,8 oder 0,2 Mol Chinolin bei pH = 8,3 verrührt, d. h. die Lobry de Bruyn und van Eckenstein-Umlagerung gemäß nachstehendem Formelschema ausführt (vgl. P. Karrer, Lehrbuch der Organischen Chemie, 13. Auflage, 1959, S. 368) :

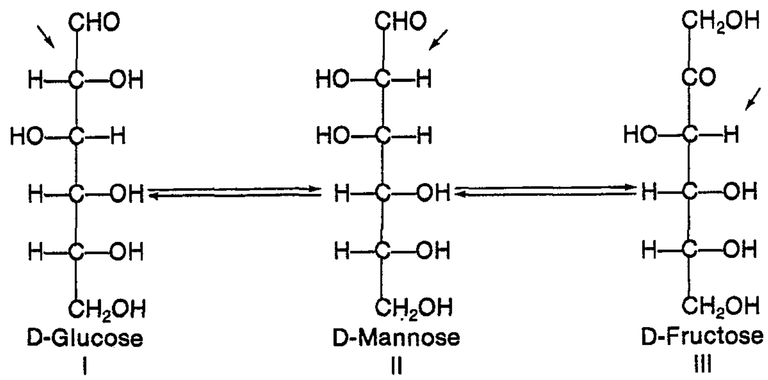

D-Glucose I — D-Mannose II — D-Fructose III

Hierbei stellen sich Gleichgewichte zwischen den Monosacchariden I, II und III ein. Anschließend führt man gemäß Beispiel 1, Variante B, die α-Addition des Formaldehyds an den bei I, II und III durch Pfeilen gekennzeichneten Stellen bei pH = 8,3 mit Dimethylcyclohexylamin als Katalysator durch. Carbonyläquivalent des Verfahrensproduktes, bezogen auf wasserfreies $C_7$-Zuckergemisch : 0,45.

In gleicher Weise kann man auch an Fehling'sche Lösung reduzierenden Mono- und Disacchariden mit Aminosäuren bzw. mit primären Aminen in erster Stufe die Amadori-Umlagerung, die Heyns-Umlagerung oder Maillard-Reaktion und anschließend in zweiter Stufe die erfindungsgemäße C-Methylolierung mit Formaldehyd durchführen. Dabei werden ebenfalls verzweigte neue Zucker erhalten, die mit Phenylhydrazin lediglich zur Phenylhydrazonbildung befähigt sind.

Beispiel 11

Das folgende Beispiel zeigt die Durchführbarkeit des erfindungsgemäßen Verfahrens an natürlichen und künstlichen Invertzuckern und technisch infolge ihrer Preiswürdigkeit besonders interessanten Isosirupen aus Maisstärke- bzw. Kartoffelstärke-Hydrolysaten, die im wesentlichen aus 1 Mol Glucose und 1 Mol Fructose bestehen. Man verfährt genau wie in Beispiel 1, Variante B, und verwendet :

a) 6 Mol eines natürlichen Invertzuckers (Bienenhonig), der im wesentlichen aus D-Glucose und D-Fructose besteht, mit einem Wassergehalt von 19,1 %, einer Viskosität von 11 848 m.Pa.s. bei 20 °C und einem Carbonyläquivalent von 0,55, berechnet auf 100 g wasserfreies Produkt.

b) 6 Mol eines aus Maisstärke bzw. Kartoffelstärke durch enzymatische Hydrolyse hergestellten « Isosirups » mit einem Wassergehalt von 15,2 % und einer Viskosität bei 25 °C von 26 169 m.Pa.s mit einem Carbonyläquivalent von 0,53, berechnet auf 100 g wasserfreies Produkt.

c) 6 Mol eines künstlichen Invertzuckers, mit einem Wassergehalt von 20 %, der aus 3 Mol Saccharose ( = Rohrzucker) durch 7 stündige Hydrolyse mit einem stark sauren Ionenaustauscher bei 70 °C

hergestellt wurde.

Carbonyläquivalent : 0,5, berechnet auf 100 g wasserfreies Produkt.

Die Reaktionsführung und Aufarbeitung erfolgt genau wie in Beispiel 1 beschrieben, d. h. es werden nur 7,1 Mol Formaldehyd verwendet, so daß auch an der Fructose lediglich C-Monomethylolierung eintritt.

Gefundene Carbonyläquivalente pro 100 g wasserfreies Verfahrensprodukt :

a) 0,43
b) 0,44
c) 0,41

## Beispiel 12

Man verfährt wie in Beispiel 11, Variante b) und verwendet für 6 Mol des im wesentlichen aus D-Glucose und D-Fructose bestehenden Gemisches 13 Mol Formaldehyd, so daß der D-Fructose-Anteil dreimal α,α'-C-methyloliert werden kann. In der Katalyse, Reaktionstemperatur, pH-Führung und Aufarbeitung verfährt man wie in Beispiel 1, Variante B).

Eine 20 %ige wäßrige Lösung der α-aldolisierten Glucose und im wesentlichen dreimal C-methylolierten Fructose besitzt bei 25 °C eine Viskosität von 24 800 m.Pa.s. Das gefundene Carbonyläquivalent beträgt 0,34, berechnet auf 100 g wasserfreie Mischung.

Berechnetes Carbonyläquivalent von 100 g einer Mischung aus 1 Mol methylolierter D-Glucose und 1 Mol trimethylolierter D-Fructose : 0,37.

Im Mittel wird also tatsächlich die D-Glucose einmal C-methyloliert, während der D-Fructose-Anteil im Isosirup etwa dreimal α,α'-methyloliert wird.

Die Acetylierung dieser $C_7$-$C_9$-Zuckergemische führt nach der im Beispiel 1 angeführten Methode zu kristallisierten, chloroformlöslichen Acetylderivaten.

## Beispiel 13

Das folgende Beispiel zeigt die Durchführung des erfindungsgemäßen Verfahrens an Disacchariden, die Fehling'sche Lösung reduzieren und zwar an

a) Maltose, Fp. : 102,5 °C
b) Lactose, Fp. : 201,6 °C
c) Cellobiose, Fp. : 225 °C

Man verfährt genau wie in Beispiel 1, Variante B, beschrieben, verwendet aber lediglich jeweils 17,1 Teile der Disaccharide (0,05 Mol) und jeweils 3 Teile Formaldehyd (0,1 Mol) und 0,3 Teile Dimethylcyclohexylamin als Katalysator. Reaktionstemperatur : 82 °C, pH-Führung : 8,3 bis 8,5.

Die gefundenen Carbonyläquivalente der α-C-methylolierten Disaccharide betragen :

a) 0,26
b) 0,25
c) 0,24

Die berechneten Carbonyläquivalente für α-C-methylolierte Maltose, Lactose und Cellobiose betragen 0,27.

Die Verfahrensprodukte a), b) und c) geben mit Phenylhydrazin praktisch in quantitativer Ausbeute Hydrazone und keine Osazone, da, wie gefunden wurde, die tert. Hydroxylgruppe am C-Atom 2 die Osazon-Bildung verhindert.

Die Trisaccharide ($C_{18}H_{32}O_{16}$) Raffinose, die in der Melasse angereichert ist, Gentianose und Melecitose, das Tetrasaccharid Stachyose, die sich im Samen vieler Leguminosen findet und das Pentasaccharid Verbascose reduzieren Fehling'sche Lösung nicht und sind der erfindungsgemäßen α-C-Methylolierung erst dann zugänglich, wenn sie z. B. in Form eines Eintopfverfahrens, zunächst der totalen oder partiellen Hydrolyse z. B. gemäß Beispiel 11, Variante c, in Galactose, Glucose und Fructose bzw. Fructose und Gentiobiose bzw. Glucose und Fructose bzw. Galactose, Glucose und Fructose unterworfen werden.

## Beispiel 14

Dieses Beispiel zeigt die erfindungsgemäße α-bzw. α,α'-C-Methylolierung von pflanzlichen Hydrolysaten bzw. von Hydrolysaten von Biomassen wie z. B. von Bäckereihefen, Nährhefen oder Bierhefen, d. h. von Hefearten, die die alkoholische Gärung von D-Glucose, D-Fructose etc. bewirken.

a) 165 Teile feuchtes, frisch geschnittenes Gras (Trockengewicht : 32 Teile) werden unter Druck bei 130 °C in 300 Teilen Wasser unter Zusatz von 0,4 Teilen Schwefelsäure im Verlaufe von 6 Stunden im Autoklaven hydrolysiert. Dabei werden die verschiedensten Polysaccharid-Reservestoffe, zuckerartigen Zellinhaltsstoffe, Cellulosen und Hemicellulosen, Proteine sowie Ribose und Desoxyribose enthaltenden Nucleinsäuren partiell hydrolysiert und in wasserlösliche Mono- und Oligosaccharide überführt. Nach Filtration, Entfernung der Schwefelsäure und aus dem Pflanzenmaterial stammender Säuren wie Phosphorsäuren etc. durch einen handelsüblichen basischen Ionenaustauscher und Einengen im Vakuum erhält man eine sirupöse Flüssigkeit. Die in ihr enthaltenen Anteile an hydrolysierten Aminosäuren bewirken im Verlaufe des Eindunstungsprozesses durch ihre Reaktion mit Keto- und Aldehydfunktionen der hydrolysierten Polysaccharide die Bildung gelbbrauner Produkte über ablaufende Maillard-Reaktionen. Ausbeute : 17 Teile ; Carbonyläquivalent : 0,38

b) Man verfährt genau wie unter a) beschrieben, verwendet aber eine feuchte, ungetrocknete Bäckereihefe (150 Teile). Ausbeute : 19 Teile eines braunen Sirups. Carbonyläquivalent : 0,41

Die gemäß a) und b) erhaltenen sirupösen Gemische enthalten ein breites Spektrum der verschiedensten Mono- und Oligosaccharide.

Man methyloliert die Zuckergemische a) und b) nach den Angaben des Beispiels 1, Variante B, und zwar mit 20 Teilen einer 30 %igen Formalinlösung (0,2 Mol Formaldehyd), verwendet 1,06 Teile Dimethylcyclohexylamin als Katalysator und führt die C-Methylolierung bei 85 °C im Verlaufe von 80 Minuten durch.

Gefundenes Carbonyläquivalent bei a), bezogen auf 100 g wasserfreies Produkt : 0,33

Gefundenes Carbonyläquivalent bei b), bezogen auf 100 g wasserfreies Produkt : 0,35

## Beispiel 15 (Verwendungsbeispiel)

Dieses Beispiel zeigt, daß durch einfaches Abmischen der neuen $C_7$-Zucker mit Aminoplastbildnern wie N-Methylolcaprolactam, ε-Caprolactam, Harnstoff, Thioharnstoff, Dicyandiamid, etc., Phenoplastbildnern wie Phenol oder Dimethylphosphit und Diäthylphosphit extrem starke Viskositätsverminderungen erzielt werden können. Die Gemische können deshalb bereits bei Raumtemperatur mit den verschiedensten Reaktionspartnern, z. B. auch mit Polyisocyanaten vermischt und zur Reaktion gebracht werden.

a) Eine Mischung aus 1 Mol α-C-methylolierter D-Glucose aus Beispiel 1, Variante B, und 2 Mol N-Methylolcaprolactam besitzt bei 35 °C lediglich eine Viskosität von 4 800 m Pa.s.

b) Eine Mischung des besonders preiswerten α-C-methylolierten Isosirups aus Beispiel 12 und 2 Mol ε-Caprolactam besitzt bei 35 °C eine Viskosität von lediglich 17 800 m Pa.s.

c) Eine Mischung aus 1 Mol α-C-methyloliertem künstlichem Invertzucker (hergestellt gemäß Beispiel 11 c aus Saccharose) mit 1,5 Mol Harnstoff besitzt bei 35 °C eine Viskosität von 14 500 m Pa.s.

d) α-C-methylolierte Glucose, z. B. aus Beispiel 1, und der technisch besonders interessante α,α'-polymethylolierte Isosirup der Beispiele 11 und 12 lassen sich in beliebigen Verhältnissen mit Dimethylphosphit, Diäthylphosphit und Triäthylphosphit zu extrem niedrigviskosen Lösungen vermischen und sind über Umesterungsreaktionen unter Abspaltung der Alkoholkomponente der Phosphite bereits bei 40-50 °C durch einfaches Anlegen eines Vakuums in vielfältiger Weise zu verestern. So besitzt eine Mischung aus 1 Mol der obigen methylolierten Zuckergemische

a) mit 2 Mol Dimethylphosphit bei 25 °C lediglich eine Viskosität von 320 m Pa.s. und

b) mit 2 Mol Diäthylphosphit bei 25 °C eine Viskosität von lediglich 450 m Pa.s.

## Beispiel 16

Verwendung erfindungsgemäß hergestellter α-methylolierter Isosirupe und ihrer Modifizierungsprodukte zur Herstellung von Hartschaumstoffen :

Eine Mischung aus

100 Teilen des mit Raney-Nickel hydrierten Verfahrensproduktes des Beispiels 12b, das anschließend mit Natriumhydroxid und Propylenoxid zu einem Polyäther der OH-Zahl 480 umgesetzt wurde,

1,5 Teilen eines handelsüblichen Siliconstabilisators (Stabilisator OS 610 der Fa. Bayer AG),

0,5 Teilen Endoäthylenpiperazin,

4,5 Teilen einer flüssigen Assoziat-Mischung aus 2,5 Teilen ε-Caprolactam und 2 Gew.-Teilen Wasser und

40 Teilen Monofluortrichlormethan

wird mit 152 Teilen eines technischen Phosgenierungsprodukts von Anilin-Formaldehyd-Kondensat (NCO-Gehalt : 29 %) intensiv vermischt. Es erfolgt eine zügige, gleichmäßige Schaumbildung. Der erhaltene geschlossenzellige Schaumstoff besitzt ein Raumgewicht von 38 kg/$m^3$.

Sofern die Herstellung weitgehend offenzelliger, sehr flammwidriger Hartschaumstoffe erwünscht ist, kann man in analoger Weise die verzweigten Zucker der Beispiele 1 bis 15 direkt verschäumen, wobei Hartschaumstoffe mit Raumgewichten von 30-24 kg/$m^3$ erhalten werden.

Die hohe Flammwidrigkeit dieser Hartschaumstoffe, insbesondere aus Aminoplastmonomeren enthaltenden Mischungen, ist vermutlich auf Dehydratisierungswasser aus den Zuckern sowie auf Wasser, das durch Kondensation von Carbonylgruppen der α-methylolierten Zucker mit den Aminoplastmonomeren beim Brandvorgang entsteht, zurückzuführen.

## Beispiel 17

a) 212 Gew.-Teile einer wäßrigen, 50 %igen Lösung der in Beispiel 1, Variante A hergestellten, an Ionenaustauschern entsalzten am α-C-Atom methylolierten Glucose werden zusammen mit 80 g Raney-Nickel in einem 0,7 Liter-Autoklaven bei 150 bar Wasserstoffdruck 4 Stunden bei 30 °C, dann 1 Stunde bei 60 °C und schließlich 1 Stunde bei 100 °C hydriert.

Man erhält eine leicht gelbliche Lösung eines neuen, 7 Hydroxylgruppen enthaltenden Polyalkohols der Konstitution

```
        CH2OH
         |
  HOH2C-C-OH
         |
       (HC-OH)3
         |
        CH2OH
```

Der neue verzweigte Polyalkohol besitzt 3 primäre reaktive Hydroxylgruppen und ist beispielsweise bei Isocyanat-Reaktionen ein wesentlich reaktiverer Vernetzer als Sorbit. Der neue Zucker neigt nicht zur Durchkristallisation. Während Sorbit mit einem Mol Kristallwasser bei ca. 100 °C schmilzt, ist der erfindungsgemäße neue Polyalkohol mit einem Mol Kristallwasser (= ca. 5,6 Gew.-% $H_2O$) bei 50 °C honigartig viskos und läßt sich bei 50 °C bereits mit anderen Polyalkoholen mischen und als Vernetzer für Isocyanatreaktionen oder Feuchthaltemittel verwenden, während dies bei Sorbit nicht möglich ist. Restcarbonylgruppengehalt : 0,016 %.

b) Eine völlige Beseitigung des Restcarbonylgruppengehaltes ist auf folgende Weise möglich : In einem 3 Liter-Edelstahlautoklaven werden 100 g Katalysator (Raney-Ni/Fe im Verhältnis 85 : 15), suspendiert in 1 Liter Wasser, vorgelegt und auf Hydriertemperatur (150 °C) aufgeheizt. Dann wird das überstehende Volumen mit Wasserstoffgas bis zum Betriebsdruck von 150 bar gefüllt. 500 ml einer gemäß Beispiel 1, Variante A hergestellten, 50 %igen wäßrigen Lösung der α-C-methylolierten Glucose werden im Verlaufe von 7 Minuten in den Autoklaven eingepumpt.

Dann läßt man 500 ml der hydrierten Lösung über ein Steigrohr mit Fritte, welche den Katalysator zurückhält, ab, pumpt die nächste Charge zu und hydriert sie wie die erste. Mit 5 weiteren Chargen von je 500 ml wird in gleicher Weise verfahren. Ein Katalysatorverlust ist nach dieser Zyklenzahl nicht festzustellen. Die hydrierten $C_7$-Polyalkohol-Lösungen werden gesammelt, über Ionenaustauscher entsalzt und im Dünnschichtverdampfer von der Hauptmenge an Wasser befreit. Man erhält einen lediglich leicht gelbstichig gefärbten $C_7$-Polyalkohol mit folgenden Eigenschaften :

Rest-Wassergehalt : 5,6 %
Rest-Carbonylgehalt : Nicht nachweisbar
OH-Zahl : 1860

Das Verfahrensprodukt ist ein hochviskoser, nicht kristallisierender verzweigter $C_7$-Polyalkohol guter Mischbarkeit mit Äthylenglykol und durch Zusatz von Äthylenglykol auf OH-Funktionalitäten von 6-2,5 einstellbar.

c) Man verfährt wie unter b) beschrieben und führt eine diskontinuierliche Hydrierung mit Ruthenium als Katalysator durch.

In einem 3 Liter Rührautoklaven werden 45 g Katalysator (5 % Ruthenium auf Kohle), suspendiert in 500 g Wasser, bei 125 °C und 200 bar $H_2$ innerhalb von 60 Minuten durch Hydrieren aktiviert. Nach Abkühlen der Suspension werden 1 500 g einer 30 %igen wäßrigen Lösung eines vollentsalzten verzweigten $C_7$-Zuckers des Beispiel 1, Variante A, (pH = 6) zugegeben. Bei einem Anfangsdruck von 150 bar $H_2$ wird das Gemisch über 30 Minuten auf die gewünschte Temperatur von 125 °C erwärmt. Bei 125 °C und 200 bar $H_2$ wird noch 90 bis 120 Minuten weiterhydriert. Als Hydrierprodukt erhält man eine farblose Lösung von $C_7$-Polyalkoholen, welche nach Abfiltrieren vom Katalysator zu einer sirupartigen Masse im Vakuum eingeengt wird.

Ausbeute : 500 Gew.-Teile, enthaltend 5,5 Gew.-% Wasser.

## Beispiel 18

212 Gew.-Teile des bei 60 °C gut rührbaren, verzweigten $C_7$-Polyalkohols nach Beispiel 17 können mit 3 Mol Essigsäureanhydrid unter Zusatz von 0,4 Gew.-Teilen Natriumacetat partiell an den primären Hydroxylgruppen acetyliert werden. Man erhält ein mit Polyestern und Polyäthern gut mischbaren, partiell acetylierten, verzweigten Polyalkohol der idealisierten Konstitution

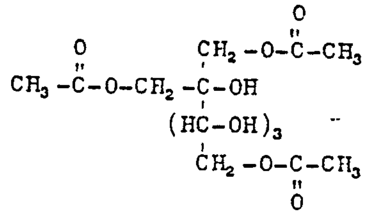

$\eta_{45\,°C} = 2\,800$ cP

der bei Verwendung als Vernetzer in Polyurethanhartschaumstoffen seine elastifizierenden Eigenschaften überträgt und die Sprödigkeit hochvernetzter Schaumstoffe verringert.

Beispiel 19

a) 224,6 Gew.-Teile des in Beispiel 1, Variante A, hergestellten verzweigten $C_7$-Zuckers mit einem Wassergehalt von 6,5 Gew.-% werden unter Stickstoffatmosphäre mit je 1,5 g eines Komplexes aus 1 Mol Bortrifluorid und 1 Mol Essigsäure bei Raumtemperatur unter Rühren vermischt. Der Ansatz wird unter Stickstoffatmosphäre gut gerührt und 406 Gew.-Teile ( = 7 Mol) Propylenoxid bei 49 °C im Verlauf von 2 Stunden gleichmäßig und langsam zugetropft. Nachdem mit Natronlauge oder wäßriger 25 %iger Ammoniaklösung ein pH-Wert von 7,2 eingestellt wurde, wird der Ansatz bei 50 °C im Vakuum von Spuren an Propylenoxid und kleinen Mengen an Wasser befreit. Man erhält verzweigte Polyäther mit überraschend niedriger Viskosität und geringem Anteil an reduzierend wirkenden Gruppen.

Ausbeute : 612 g ; OH-Zahl : 635 ; Säurezahl 0,7 ; Viskosität 16 000 mPas/35 °C.

Der geringe Gehalt an reduzierend wirkenden Zuckeranteilen von lediglich 3,1 % zeigt, daß bei der Propoxylierung überrasenderweise die Carbonylgruppen in den Verfahrensprodukten in erheblichem Umfang acetalisiert wurden. Es werden daher über 90 % der im verzweigten $C_7$-Zucker vorliegenden Aldehydgruppen acetalisiert.

Die neuen, verzweigten Polyäther sind mit hoch- und niedermolekularen Polyhydroxylverbindungen, aber auch mit Isocyanaten besser verträglich als entwässerte Ausgangspolyalkohole. Von besonderer Bedeutung ist ferner, daß die erhaltenen verzweigten Polyäther bei ihrer Reaktion mit Polyisocyanaten wesentlich aktiver sind als üblicherweise durch $OH^{\ominus}$-Katalyse in Gegenwart von Natronlauge oder Kalilauge hergestellte Formose-Polyäther.

c) Setzt man 224,6 Gew.-Teile des in Beispiel 17, Variante b, hydrierten Polyalkohols ein und verfährt genau wie bei a), erhält man einen nahezu farblosen Polyäther in einer Ausbeute von 616 g, der völlig frei von Dioxolaneinheiten und reduzierend wirkenden Anteilen ist.

OH-Zahl 650.

Beispiel 20

2 240 g der in Beispiel 17, Variante b) beschriebenen mehrwertigen verzweigten Polyalkohols und 600 g Toluol werden bei Raumtemperatur vorgelegt. Durch zweimaliges Evakuieren und Wiederauffüllen des Reaktionsgefäßes mit Stickstoff wird der Luftsauerstoff entfernt. Nach Erwärmen auf 80 °C werden 80 g 50 %ige wäßrige Kalilauge zugegeben. Anschließend wird weiter erwärmt. Zwischen 100-115 °C werden 52,8 g Wasser (Lösungswasser und Reaktionswasser aus der Kalilauge) azeotrop abdestilliert. Nach beendeter Destillation werden zu dem sehr gut rührbaren Gemisch bei 100-105 °C und bei einem Druck von 0,4 bis 0,6 bar 6 112 g Propylenoxid allmählich zudosiert (500 g/Stunde). Je nach Erfordernis wird durch Kühlen oder Heizen des Reaktionsgemisches die Reaktionstemperatur in diesem Bereich gehalten. Nach beendeter Propylenoxidzugabe wird noch weitere 3 Stunden bei 100-105 °C gerührt.

Das alkalische Polymerisat wird nach Zugabe von 800 g Wasser mit 284 g 12,5 %iger wäßriger Schwefelsäure neutralisiert (pH-Wert der Emulsion 6,8). Anschließend wird bei 70 bis 90 °C nach Zugabe von Filtrierhilfsmitteln (Zellstoffpulver und synthetisches Magnesiumsilikat) und einem Antioxidans (2,6-Di-tert.-butyl-p-kresol) das Wasser im Vakuum abdestilliert. Bei einem Wassergehalt von 0,9 % werden die abgeschiedenen Salze und die Filtrierhilfsmittel abfiltriert.

Zur vollständigen Entfernung des Wassers wird das Filtrat anschließend bei 100-105 °C im Vakuum ausdestilliert.

Das erhaltene nahezu farblose Produkt hat die folgenden physikalischen Daten :

| | |
|---|---|
| Hydroxylzahl (mg KOH/g) | 300 |
| pH-Wert | 7,6. |
| Wassergehalt (%) | 0,05 |
| Viskosität 25 °C (mPas) | 5 440 |

Das auf diese Weise erhaltene Polyätherpolyol kann zu einem harten Polyurethanschaumstoff verarbeitet werden. Bedingt durch die geringe Viskosität des erhaltenen Polyäthers ergibt sich ein

verbessertes Fließvermögen des Reaktionsgemisches gegenüber handelsüblichen Saccharose-Polyäthern.

Beispiel 21

424 Gew.-Teile des in Beispiel 6, Variante e), durch NaCN-Katalyse erhaltenen fast farblosen verzweigten $C_7$-Zuckers werden mit 1 Mol Oxalsäure bereits bei 60 °C in rascher Veresterungsreaktion und ohne nennenswerte Dehydratisierung bevorzugt an ihren primären Hydroxylgruppen unter Abspaltung von 36 Gew.-Teilen Kondensationswasser verestert. Man erhält einen Polyester der idealisierten Konstitution

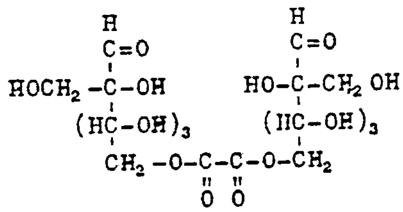

in einer Ausbeute von 456 Gew.-Teilen, der in Polyestern von Adipinsäure und Diäthylenglykol vom Durchschnittsmolekulargewicht 2 000 zu 30 Gew.-% gelöst werden kann, während ein vergleichbares Glucose-Oxalsäure Kondensat im gleichen Polyester nicht mischbar ist.

Beispiel 22

a) 200 g des in Beispiel 17, Variante b) beschriebenen, verzweigten mehrwertigen Polyalkohols werden im Dünnschichtenverdampfer bei 100 °C und 0,06 Torr entwässert und mit 0,5 g Triäthylendiamin versetzt.

Die Mischung wird auf 100 °C erwärmt. Bei dieser Temperatur werden innerhalb von 40 Minuten 281 g Stearylisocyanat zugetropft und die Mischung so lange nachgerührt, bis mit Hilfe der IR-Spektroskopie kein Isocyanat mehr nachzuweisen ist. Nach dem Abkühlen erhält man ein wachsartiges Produkt mit guten oberflächenaktiven Eigenschaften, das einen vorzüglichen Emulgator zur Emulgierung von Wasser in Polyisocyanaten darstellt.

b) 424 g des verzweigten, mehrwertigen Polyalkohols (wie bei a) werden bei 100 °C bei 0,06 Torr entwässert. Das wasserfreie Gemisch wird mit 1 600 g Dimethylformamid und 562 g Stearinsäuremethylester versetzt. Zu der Mischung werden bei Raumtemperatur 70 g einer 30 %igen Natriummethylatlösung zugetropft und die Mischung anschließend bei 95-100 °C und 180 Torr solange gerührt, bis kein Methanol mehr abdestilliert.

Nach Abdestillieren des Dimethylformamids erhält man eine wachsartige Masse, die durch Behandeln mit heißem Wasser von überschüssigem Polyalkoholgemisch befreit wird. Die wäßrige Aufschlämmung wird vom überschüssigen Wasser abgepreßt und im Vakuum getrocknet. Man erhält eine wachsartige Masse mit guten oberflächenaktiven Eigenschaften, die einen guten Emulgator zur Emulgierung von Wasser und wasserlöslichen Polyalkoholen in hydrophobe Polyäther darstellt.

Beispiel 23

a) Gemäß der Verfahrensweise der DE-OS 2 031 160 und DE-OS 1 953 347 (Imprägnierungsreaktionen) imprägniert man einen hydrophilen Polyurethan-Weichschaumstoff-Quader der Dimensionen 20 cm × 10 cm ×5 cm = 1 000 $cm^3$ Volumen, der in seinem Polyäther-Segmentanteil ca. 40 Gew.-% Polyäthylenoxid-Segmente enthält, mit einer 50 %igen wäßrigen Lösung eines $C_7$-verzweigten Alkohols, der gemäß Beispiel 1, Variante A und nachfolgender Hydrierung hergestellt wurde, wobei der Schaumstoff spontan gleichmäßig in den Dimensionen des Raumes stark quillt. Die erreichte Volumvergrößerung des Quaders beträgt nach dem Ausquetschen des Imprägnierungsmittels und Trocknen bei 30 °C im Vakuum 2 000 $cm^3$, d. h. durch Imprägnierung und Quellung ist ein $\Delta V$ von etwa 1 000 $cm^3$ erzeugt worden. Der so beladene Schaumstoff enthält ca. 30 Gew.-Teile an verzweigten hydrierten $C_7$-Zuckern und es werden ca. 12 Gew.-Teile Wasser durch das verwendete Feuchthaltemittel fixiert. Die starke Quellung des Schaumstoffs wird durch Solvation der Weichsegmente des Schaumstoffes bedingt. Bei langen Lagerzeiten von 2 Monaten wird das Wasser nicht abgegeben. Der Schaumstoff behält seinen wesentlich weicheren Griff gegenüber dem eingesetzten, nichtimprägnierten Schaumstoff. Folien aus derartigen, durch Quellvorgänge mit den erfindungsgemäßen Feuchthaltemitteln in ihrem Volumen vergrößerte Schaumstoffe besitzen einen sehr weichen, klebfreien Griff und eignen sich insbesondere als Wischtücher zur Verhinderung der Vereisung von Autofensterscheiben bei Minustemperaturen von ca. − 10 °C.

b) Man verfährt wie bei a) und dispergiert vor der Imprägnierung

1) 20 Gew.-Teile an handelsüblicher Nivea-Creme in der erfindungsgemäßen Zuckerlösung
2) 20 Gew.-Teile einer handelsüblichen Schuhcreme in der erfindungsgemäßen Zuckerlösung.

Die unter 1) und 2) genannten Zusatzmittel trocknen selbst nach 3 Monaten bei Lagerung an der Luft (22-25 °C) nicht ein.

Beispiel 24

100 Gew.-Teile eines 6 Gew.-% Wasser enthaltenden Zuckers der Konstitution

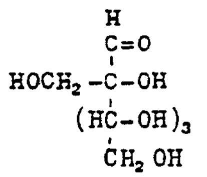

( = ca. 0,45 Mol)

werden in 900 g Wasser gelöst. Der Ansatz wird mit 1 g Ammoniumcarbonat, 1 g prim. Kaliumphosphat und 100 g feuchter Bäckereihefe versetzt ( = 20 g Hefetrockengewicht). Unter Stickstoffatmosphäre wird die Suspension gerührt. Die Hefe befindet sich in Zellteilung, vermehrt sich und gleichzeitig läuft die enzymatische partielle Vergärungsreaktion ein. Entwickeltes $CO_2$ wird durch Überleiten eines Stickstockstromes über die gerührte Suspension in einer 1 n-NaOH-Lösung absorbiert, $CO_2$ nach der Bariumcarbonatmethode bestimmt und die Gärungskurve ($CO_2$-Abspaltung als Funktion der Zeit) graphisch aufgetragen.

Nach 4,5 Stunden ist die $CO_2$-Entwicklung praktisch beendet und es sind 18,5 g $CO_2$ entbunden und 20,6 g Äthanol entstanden.

Die rechnerische Auswertung führt zu dem Ergebnis, daß die neuen verzweigten $C_7$-Zucker ( = α-C-methylolierte Glucose) zur Hälfte im unteren Molekülteil enzymatisch gespalten wird

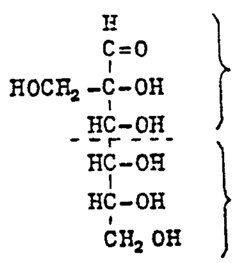

mit Harnstoff kondensationsfähiger Molekülteil

« vergärbarer Molekülteil »

0,45 Mol des abgespaltenen Polyhydroxyaldehyd-Restes

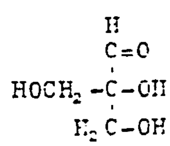

kondensieren mit 20 g zugesetztem Harnstoff ( = 0,33 Mol) zu einem orangefarbenen Sirup.
(Ausbeute : 71 g).

**Ansprüche**

1. Umsetzungsprodukte natürlicher, Fehling'sche Lösung reduzierender $C_6$-Zucker und ihrer Oligomeren mit einem Molekulargewicht bis zu 2 000, dadurch gekennzeichnet, daß sie in α- und/oder α'-Stellung zur Carbonylgruppe bzw. Cyclohalbacetalgruppe des $C_6$-Zuckers und ihrer Oligomeren mindestens eine, das Kohlenstoffgerüst des $C_6$-Zuckers und ihrer Oligomere verzweigende Methylolgruppe aufweisen.

2. Flüssige Gemische aus
   a) 95 bis 5 Gew.-% der Verbindungen gemäß Anspruch 1 und
   b) 5 bis 95 Gew.-% Glucose und/oder Saccharose.

3. Flüssige Gemische aus
   a) 99 bis 5 Gew.-% an Verbindungen gemäß Anspruch 1 und
   b) 1 bis 95 Gew.-% an Alkylphosphiten und/oder Aminoplastmonomeren und/oder Phenoplastmonomeren und/oder in α-Stellung zur Carbonylgruppe mindestens eine Methylolgruppe aufweisenden

niedermolekularen Aldehyden und/oder Ketonen.

4. Verfahren zur Herstellung von Umsetzungsprodukten von $C_6$-Zuckern und ihrer Oligomeren mit einem Molekulargewicht bis zu 2 000 gemäß Anspruch 1, dadurch gekennzeichnet, daß man einen Fehling'sche Lösung reduzierenden natürlichen $C_6$-Zucker oder ein Oligomeres von $C_6$-Zuckern bzw. ein Fehling'sche Lösung reduzierendes Derivat eines natürlichen $C_6$-Zuckers oder ihrer Oligomeren, gegebenenfalls in Anwesenheit von Wasser und/oder ein- oder mehrwertigen Alkoholen mit einem Molekulargewicht zwischen 32 und 10 000 und/oder zur Bildung von Aminoplasten oder Phenoplasten geeigneten Stoffe und/oder nicht zuckerartigen Aldehyden und Ketonen und/oder Alkylphosphiten, bei einer Temperatur von 40 bis 110 °C und bei einem pH-Wert von 7,4 bis 11 mit 0,05 bis 10 Mol, bezogen auf die Äquivalente an zur Carbonylgruppe bzw. Cyclohalbacetalgruppe des $C_6$-Zuckers oder eines Oligomeren von $C_6$-Zuckern $\alpha$- und $\alpha'$-ständigen Wasserstoffatomen, an Formaldehyd umsetzt.

5. Verfahren nach Anspruch 4, dadurch gekennzeichnet, daß als Formaldehydquelle ein Formaldehydgas enthaltendes Synthesegas eingesetzt wird, wobei man das Synthesegas diskontinuierlich durch eine ruhende oder kontinuierlich durch eine im Gegenstrom bewegte wäßrige und/oder alkoholische Lösung des zu methylolierenden Zuckers leitet, welche gegebenenfalls Alkylphosphite und/oder Aminoplastomonomere und/oder Phenoplastomonomere und/oder zusätzlich niedermolekulare, zur $\alpha$-Methylolierung befähigte Oxoverbindungen enthält, und wobei man die Methylolierungsreaktion in Gegenwart von 0,04 bis 0,06 Mol, bezogen auf Aldehyd- oder Keto-Äquivalente des Zuckers, an organischen und/oder anorganischen Basen durchführt, die vor, während oder nach der Absorption zugegeben werden, so daß die Methylolierung simultan zur Absorption oder teilweise oder ganz nach der Absorption, gegebenenfalls in einem nachgeschalteten Reaktorsystem, erfolgt.

6. Verfahren nach Anspruch 4 und 5, dadurch gekennzeichnet, daß man als Base zur Einstellung des pH-Wertes tertiäre Amine, quartäre Ammoniumbasen oder Alkalimetallcyanide verwendet.

7. Verfahren nach Anspruch 4 und 5, dadurch gekennzeichnet, daß man den nach der Methylolierungsreaktion vorhandenen Restformaldehyd durch Zusatz von Aminoplast- und/oder Phenoplastbildenden Monomeren und/oder primären oder sekundären Aminen und/oder Alkylphosphiten oder durch Ansäuern auf pH-Werte von 1 bis 3 unter intra- bzw. intermolekularer Acetalbildung bindet.

8. Verwendung von Umsetzungsprodukten von $C_6$-Zuckern und ihren Oligomeren mit einem Molekulargewicht bis zu 2 000 gemäß Anspruch 1 zur Herstellung von gegebenenfalls zellförmigen Polyurethankunststoffen durch Umsetzung von

A) Polyisocyanaten mit

B) Polyhydroxylverbindungen sowie gegebenenfalls

C) weiteren, mindestens zwei aktive Wasserstoffatome aufweisenden Verbindungen mit einem Molekulargewicht zwischen 62 und 10 000, gegebenenfalls in Anwesenheit von

D) Treibmitteln, Katalysatoren und weiteren an sich bekannten Zusatzstoffen

dadurch gekennzeichnet, daß als Komponente B Umsetzungsprodukte von $C_6$-Zuckern bzw. Ihrer Oligomeren mit einem Molekulargewicht bis zu 2 000 gemäß Anspruch 1 eingesetzt werden, wobei gegebenenfalls ein Teil oder die Gesamtmenge dieser Umsetzungsprodukte von $C_6$-Zuckern bzw. ihrer Oligomeren zunächst hydriert und/oder oxalkyliert werden und dann als Komponente B eingesetzt werden.

**Claims**

1. Reaction products of natural $C_6$ sugars and oligomers thereof having a molecular weight of up to 2,000 which reduce Fehling's solution, characterised in that they have at least one methylol group wich branches the carbon structure of the $C_6$ sugars and oligomers thereof in the $\alpha$- and/or $\alpha'$-position to the carbonyl group or cyclohemiacetal group of the $C_6$ sugars and oligomers thereof.

2. Liquid mixtures of

a) 95 to 5 % by weight of the compounds according to Claim 1 and

b) 5 to 95 % by weight of glucose and/or saccharose.

3. Liquid mixtures of

a) 99 to 5 % by weight of compounds according to Claim 1 and

b) 1 to 95 % by weight of alkyl phosphites and/or aminoplast monomers and/or phenoplast monomers and/or low molecular aldehydes and/or ketones which have at least one methylol group in the $\alpha$-position to the carbonyl group.

4. Process for the preparation of reaction products of $C_6$ sugars and oligomers thereof having a molecular weight of up to 2,000, according to Claim 1, characterised in that natural $C_6$ sugars or oligomers of $C_6$ sugars which reduce Fehling's solution, or derivatives of natural $C_6$ sugars or oligomers thereof which reduce Fehling's solution, are reacted with 0.05 to 10 mol, based on the equivalents of the hydrogen atoms in the $\alpha$- and $\alpha'$-position to the carbonyl group or cyclohemiacetal group of the $C_6$ sugars or oligomers thereof, of formaldehyde, at a pH value of 7.4 to 11 and a temperature of 40 to 110 °C, optionally in the presence of water and/or monohydric or polyhydric alcohols having a molecular weight of between 32 and 10,000 and/or substances suitable for the formation of aminoplasts or phenoplasts and/or non-sugar-like aldehydes and ketones and/or alkyl phosphites.

5. Process according to Claim 4, characterised in that the source of formaldehyde used is a synthesis gas containing formaldehyde gas, which synthesis gas is passed discontinuously through a stationary or continuously through a countercurrently moving aqueous and/or alcoholic solution of the sugars to be methylolated, which solution may contain alkyl phosphites and/or aminoplast monomers and/or phenoplast monomers and/or, in addition, low molecular oxo compounds which are capable of α-methylolation, the methylolation reaction being carried out in the presence of 0.04 to 0.06 mol, based on the aldehyde or keto equivalents of the sugars, of organic and/or inorganic bases, which are added before, during or after the absorption, so that the methylolation takes place simultaneously to the absorption or partly or completely after the absorption, if appropriate is a subsequent reactor system.

6. Process according to Claim 4 and 5, characterised in that tertiary amines, quaternary ammonium bases or alkali metal cyanides are used as the base for adjusting the pH value.

7. Process according to Claim 4 and 5, characterised in that the residual formaldehyde present after the methylolation reaction is bound by the addition of aminoplast- and/or phenoplast-forming monomers and/or primary or secondary amines and/or alkyl phosphites or by acidification to pH values of 1 to 3, accompanied by intra- or inter-molecular acetal formation.

8. Use of reaction products of $C_6$ sugars and oligomers thereof having a molecular weight of up to 2, 000, according to Claim 1, for the production of optionally cellular polyurethane plastics by reacting

A) polyisocyanates with

B) polyhydroxyl compounds and optionally

C) other compounds having a molecular weight of between 62 and 10,000 which have at least two active hydrogen atoms, optionally in the presence of

D) blowing agents, catalysts and other known additives, characterised in that reaction products of $C_6$ sugars or oligomers thereof having a molecular weight of up to 2,000, according to Claim 1, are used as component B, a part or the total quantity of these reaction products of $C_6$ sugars or oligomers thereof being, if appropriate, first hydrogenated and/or alkoxylated and then used as component B.

**Revendications**

1. Produits de réaction de sucres naturels en $C_6$ réduisant la liqueur de Fehling et de leurs oligomères de poids moléculaire allant jusqu'à 2 000, caractérisés en ce qu'ils portent en position α et/ou en α' par rapport au groupe carbonyle ou au groupe cyclo-hémi-acétal du sucre en $C_6$ et de ses oligomères, au moins un groupe méthylol ramifiant le squelette carboné du sucre en $C_6$ et de ses oligomères.

2. Mélanges liquides

a) de 95 à 5 % en poids des composés suivant la revendication 1 et

b) de 5 à 95 % en poids de glucose et/ou de saccharose.

3. Mélanges liquides

a) de 99 à 5 % en poids de composés suivant la revendication 1 et

b) de 1 à 95 % en poids de phosphites d'alkyle et/ou de monomères d'aminoplastes et/ou de monomères de phénoplastes et/ou d'aldéhydes et/ou de cétones de bas poids moléculaire portant au moins un groupe méthylol en position α par rapport au groupe carbonyle.

4. Procédé pour l'obtention de produits de réaction de sucres en $C_6$ et de leurs oligomères de poids moléculaire allant jusqu'à 2 000 suivant la revendication 1, caractérisé en ce qu'on fait réagir un sucre naturel en $C_6$ réduisant la liqueur de Fehling ou un oligomère de sucres en $C_6$ ou un dérivé, réduisant la liqueur de Fehling, d'un sucre naturel en $C_6$ ou de ses oligomères, éventuellement en présence d'eau et/ou d'alcools monovalents ou polyvalents de poids moléculaire compris entre 32 et 10 000 et/ou de substances qui conviennent à la formation d'aminoplastes ou de phénoplastes et/ou d'aldéhydes et de cétones n'appartenant pas au genre sucre et/ou de phosphites d'alkyle, à une température de 40 à 110 °C et à une valeur de pH de 7,4 à 11 avec 0,05 à 10 moles de formaldéhyde, relativement aux équivalents d'atomes d'hydrogène en positions α et α' par rapport au groupe carbonyle ou au groupe cyclo-hémi-acétal du sucre en $C_6$ ou d'un oligomère de sucres en $C_6$.

5. Procédé suivant la revendication 4, caractérisé en ce qu'on utilise comme source de formaldéhyde un gaz de synthèse contenant du formaldéhyde gazeux, en faisant passer le gaz de synthèse en discontinu dans une solution aqueuse et/ou alcoolique au repos du sucre à méthyloler ou en continu dans une telle solution en mouvement à contre-courant, qui contient, le cas échéant, des phosphites d'alkyle et/ou des monomères d'aminoplastes et/ou des monomères de phénoplastes et/ou, en outre, des composés oxo de bas poids moléculaire susceptibles de méthylolation en α, et on conduit alors la réaction de méthylolation en présence de 0,04 à 0,06 mole, par rapport aux équivalents aldéhydiques ou cétoniques du sucre, de bases organiques et/ou inorganiques qui sont ajoutées avant, pendant ou après l'absorption de manière que la méthylolation s'effectue en même temps que l'absorption ou en partie ou en totalité après l'absorption, éventuellement dans un réacteur intercalé à la suite.

6. Procédé suivant les revendications 4 et 5, caractérisé en ce qu'on utilise des amines tertiaires, des bases d'ammonium quaternaire ou des cyanures de métaux alcalins comme base pour ajuster la valeur du pH.

7. Procédé suivant les revendications 4 et 5, caractérisé en ce qu'on fixe le formaldéhyde résiduel présent après la réaction de méthylolation par addition de monomères formant des aminoplastes et/ou des phénoplastes et/ou d'amines primaires ou secondaires et/ou de phosphites d'alkyle ou par acidification à des pH de 1 à 3 avec formation d'acétal intramoléculaire ou intermoléculaire.

8. Utilisation de produits de réaction de sucres en $C_6$ et de leurs oligomères de poids moléculaire allant jusqu'à 2 000 suivant la revendication 1 pour la production de matières plastiques du type polyuréthanne éventuellement cellulaires par réaction

A) de polyisocyanates avec

B) des composés polyhydroxyliques ainsi que, le cas échéant,

C) d'autres composés portant au moins deux atomes d'hydrogène actif, ayant un poids moléculaire de 62 à 10 000, éventuellement en présence

D) d'agents porogènes, de catalyseurs et d'autres additifs connus,

caractérisée en ce qu'on utilise comme composant B des produits de réaction de sucres en $C_6$ ou de leurs oligomères de poids moléculaire allant jusqu'à 2 000 suivant la revendication 1, en hydrogénant et/ou en oxalkylant tout d'abord, le cas échéant, une partie ou la totalité de ces produits de réaction de sucres en $C_6$ ou de leurs oligomères, puis en les utilisant comme composant B.

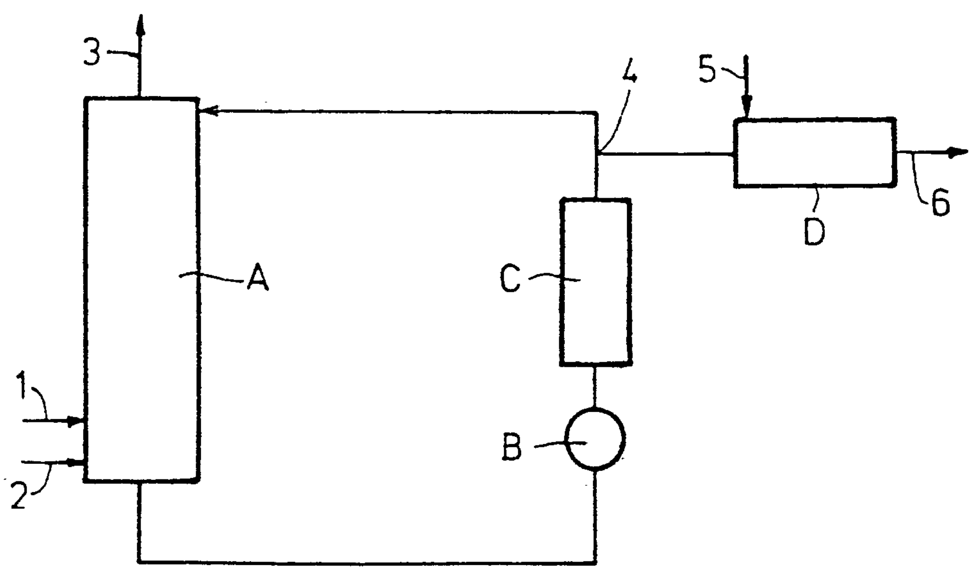


FIG. 1